(19) Europäisches Patentamt
European Patent Office
Office européen des brevets

(11) EP 4 748 396 A1

(12) EUROPEAN PATENT APPLICATION
published in accordance with Art. 153(4) EPC

(43) Date of publication:
**27.05.2026 Bulletin 2026/22**

(21) Application number: **24844764.1**

(22) Date of filing: **22.07.2024**

(51) International Patent Classification (IPC):
***A61K 45/06*** *(2006.01)* ***A61K 47/68*** *(2017.01)*
***A61K 39/395*** *(2006.01)* ***A61K 39/00*** *(2006.01)*
***A61P 35/00*** *(2006.01)*

(52) Cooperative Patent Classification (CPC):
**A61K 39/00; A61K 39/395; A61K 45/06; A61K 47/68; A61P 35/00; A61P 35/04; C07K 16/28**

(86) International application number:
**PCT/CN2024/106818**

(87) International publication number:
**WO 2025/021073 (30.01.2025 Gazette 2025/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.07.2023 CN 202310901329**

(71) Applicants:
- **Jiangsu Mabwell Health Pharmaceutical R&D Co., Ltd.**
  **China Medical City**
  **Taizhou, Jiangsu 225300 (CN)**
- **Mabwell (Shanghai) Bioscience Co., Ltd.**
  **Shanghai 201210 (CN)**

(72) Inventors:
- **YU, Dongan**
  **Taizhou, Jiangsu 225300 (CN)**
- **ZHOU, Wei**
  **Taizhou, Jiangsu 225300 (CN)**
- **FANG, Peng**
  **Taizhou, Jiangsu 225300 (CN)**
- **TAN, Xiaoding**
  **Taizhou, Jiangsu 225300 (CN)**
- **LIU, Datao**
  **Shanghai 201210 (CN)**
- **WANG, Peipei**
  **Shanghai 201210 (CN)**
- **FENG, Zhe**
  **Shanghai 201210 (CN)**

(74) Representative: **Bianchetti & Minoja with Trevisan & Cuonzo IPS SRL**
**Via Plinio, 63**
**20129 Milano (IT)**

Remarks:
The complete document including Reference Table(s) and the Sequence Listing(s) can be downloaded from the EPO website

# (54) PHARMACEUTICAL COMPOSITION CONTAINING ANTI-NECTIN4 ANTIBODY-DRUG CONJUGATE AND ANTI-PD-1 ANTIBODY AND USE THEREOF

(57) A pharmaceutical composition containing an anti-Nectin4 antibody-drug conjugate and an anti-PD-1 antibody and a use thereof in preventing or treating a cancer. The pharmaceutical composition has good safety, and has a stronger anti-tumor effect than using a drug alone. The pharmaceutical composition shows a synergistic tumor inhibitory effect. The present invention also relates to a kit of parts containing the pharmaceutical composition.

EP 4 748 396 A1

## Description

### Technical Field

The present invention relates to the field of medicine. Specifically, the present invention relates to a pharmaceutical combination comprising an anti-Nectin4 antibody-drug conjugate and an anti-PD-1 antibody or an antigen-binding fragment thereof targeting programmed death-1 (PD-1), and a use of the pharmaceutical combination for preventing or treating a cancer.

### Background Art

Nectin-4 (also known as "Poliovirus Receptor-Like Molecule 4 (PVRL4)") is a type I transmembrane glycoprotein with a molecular weight of approximately 66 KD. It belongs to a member of Nectin family within the immunoglobulin superfamily. Its extracellular domain consists of three Ig like domains (VCC type), and it cooperates with cadherins to participate in the formation and maintenance of adherens junctions.

Nectin-4 exhibits high expression in normal embryonic and fetal tissues, declines in adulthood, and shows limited distribution in healthy tissues. It is highly expressed in various tumor cells, such as colon cancer, ovarian cancer, cervical cancer, urothelial cancer, breast cancer, lung cancer, gastric cancer, and head and neck cancer. It mainly promotes the proliferation, differentiation, migration, invasion of tumor cells and the like by activating the PI3K/Akt pathway. Therefore, targeting Nectin-4 is emerging as an effective therapeutic strategy for treating cancers expressing Nectin-4.

Anti-Nectin-4 antibody-drug conjugates (anti-Nectin4 ADCs) consist of monoclonal antibodies targeting the specific antigen Nectin-4, linked to small-molecule cytotoxic drugs via linkers (also termed "connectors"). Anti-Nectin-4 antibody-drug conjugates combine the potent cytotoxic effect of traditional small-molecule chemotherapy with the tumor-targeting capability of antibody drugs, and are composed of three main components: an antibody responsible for selectively recognizing the antigen Nectin-4 on the surface of cancer cells, a drug as a payload responsible for killing cancer cells, and a linker connecting the antibody and the payload.

Although antibody-drug conjugates (ADCs) have now become a popular class of drugs for treating hematological malignancies and solid tumors, extensive preclinical and clinical studies have shown that in tumor treatment with antibody-drug conjugates, the duration of objective responses or clinical benefits achieved with ADCs as monotherapy remains limited. Therefore, combining ADCs with other anticancer drugs has become an important direction in ADC drug development.

PD-1 is a key immune checkpoint receptor expressed by activated T and B cells, and mediates immune suppression (Yao S, Zhu Y, and Chen L. Advances in targeting cell surface signaling molecules for immune modulation. Nat. Rev. Drug Discov., 2013, 12(2):130-146). Two cell surface glycoprotein ligands for PD-1 have been identified, namely programmed death-ligand 1 (PD-L1) and programmed death-ligand 2 (PD-L2). They are expressed on antigen-presenting cells and numerous human cancers. Research results in prior art demonstrate that their binding to PD-1 can induce T cell apoptosis, immune unresponsiveness, T cell "exhaustion", and IL-10 secretion and the like. Studies have demonstrated that blocking the binding of PD-1 to its ligands can restore T-cell function in cancer patients. Monoclonal antibodies targeting PD-1, such as Toripalimab, Pembrolizumab, or Pidilizumab, have been described. Upon binding to PD-1 on T lymphocytes, the anti-PD-1 monoclonal antibodies can inhibit the binding of PD-1 to its ligands, thereby promoting T lymphocyte activation and proliferation, inducing the production of immune-activating cytokines such as IL-2, and reversing the suppression of PD-1 on the immune surveillance of active T lymphocytes having anti-tumor activity.

The biological behaviors of most tumors are not governed by a single signaling pathway but rather by the combined action of multiple signaling pathways. In certain cases, drugs with different mechanisms of action can be used in combination. However, any combination of drugs that have different mechanisms of action but function in similar field does not necessarily yield a combination with beneficial effects. Therefore, although there is indeed a need in the prior art for combination therapy regimens and products targeting different signaling pathways, discovering feasible combination therapy regimens and products that can deliver superior effects (such as reducing the dose of individual drugs, improving the incidence and/or severity of adverse events (AEs) during treatment, and/or acting in a synergistic effect) compared with monotherapy remains a significant challenge in the pharmaceutical field, due to the complexity of tumorigenic mechanisms and the unpredictability of interactions between different drugs.

### Summary of the Invention

The inventors surprisingly discovered that the pharmaceutical combination of the present application can exert preventive and/or therapeutic effects on cancer in a manner that achieves synergistic effects and/or improves the incidence and/or severity of adverse events (AEs) during treatment.

The present invention provides a pharmaceutical combination comprising an anti-Nectin4 antibody-drug

conjugate or pharmaceutically acceptable salt thereof with an anti-PD-1 antibody or antigen-binding fragment thereof, and a use of the combination for preventing or treating cancers.

Specifically, the present invention relates to the following aspects.

In a first aspect, the present invention provides a pharmaceutical combination comprising

(i) an anti-Nectin4 antibody-drug conjugate or a pharmaceutically acceptable salt thereof; and

(ii) an anti-PD-1 antibody or an antigen-binding fragment thereof.

In some embodiments, the anti-PD-1 antibody or the antigen-binding fragment thereof in the pharmaceutical combination of the present invention comprises

(i) a heavy chain variable region and a light chain variable region, wherein the heavy chain variable region comprises HCDR1 shown in SEQ ID NO:11, HCDR2 shown in SEQ ID NO:12, and HCDR3 shown in SEQ ID NO:13; and the light chain variable region comprises LCDR1 shown in SEQ ID NO:14, LCDR2 shown in SEQ ID NO:15, and LCDR3 shown in SEQ ID NO:16; or

(ii) a heavy chain variable region and a light chain variable region, wherein the heavy chain variable region comprises HCDR1 shown in SEQ ID NO:21, HCDR2 shown in SEQ ID NO:22, and HCDR3 shown in SEQ ID NO:23; and the light chain variable region comprises LCDR1 shown in SEQ ID NO:24, LCDR2 shown in SEQ ID NO:25, and LCDR3 shown in SEQ ID NO:26;

Preferably, the anti-PD-1 antibody or the antigen-binding fragment thereof comprises

(i) a heavy chain variable region and a light chain variable region, wherein the heavy chain variable region comprises a sequence shown in SEQ ID NO:17 or a sequence having at least 90%, 91%, 92%, 93%, 94%, 95%, 96%, 97%, 98%, or 99% identity therewith, and the light chain variable region comprises a sequence shown in SEQ ID NO:18 or a sequence having at least 90%, 91%, 92%, 93%, 94%, 95%, 96%, 97%, 98%, or 99% identity therewith; or

(ii) a heavy chain variable region and a light chain variable region, wherein the heavy chain variable region comprises a sequence shown in SEQ ID NO:27 or a sequence having at least 90%, 91%, 92%, 93%, 94%, 95%, 96%, 97%, 98%, or 99% identity therewith, and the light chain variable region comprises a sequence shown in SEQ ID NO:28 or a sequence having at least 90%, 91%, 92%, 93%, 94%, 95%, 96%, 97%, 98%, or 99% identity therewith;

More preferably, the anti-PD-1 antibody comprises

(i) a heavy chain sequence of SEQ ID NO:19 or a sequence having at least 90%, 95%, 98%, or 99% identity therewith, and a light chain sequence of SEQ ID NO:20 or a sequence having at least 90%, 95%, 98%, or 99% identity therewith; or

(ii) a heavy chain sequence of SEQ ID NO:29 or a sequence having at least 90%, 95%, 98%, or 99% identity therewith, and a light chain sequence of SEQ ID NO:30 or a sequence having at least 90%, 95%, 98%, or 99% identity therewith.

In some embodiments, the anti-PD-1 antibody or the antigen-binding fragment thereof in the pharmaceutical combination of the present invention is an IgG antibody, preferably a human IgG antibody, more preferably a human IgG1 or human IgG4 antibody; and wherein the antigen-binding fragment is Fab, Fab', $F(ab')_2$, Fv, single-chain Fv, or single-chain Fab.

In some embodiments, the anti-Nectin4 antibody-drug conjugate or the pharmaceutically acceptable salt thereof in the pharmaceutical combination of the present invention has a molecular formula of Ab-[L-CTD]m, wherein Ab represents an anti-Nectin-4 antibody or an antigen-binding fragment thereof, L represents a linker, CTD represents a drug, and m represents an average number of drugs conjugated per Ab molecule; preferably, CTD is a cytotoxic drug; preferably CTD is one or more selected from the group consisting of: microtubule inhibitors MMAE, DM1, DM4, Tublysin, amanitin, calicheamicin, Eribulin and derivatives thereof; topoisomerase inhibitors SN38, Exatecan and derivatives thereof; and DNA binding agents PBD, doxorubicin and derivatives thereof; preferably, m is 1.0 to 5.0, preferably 3.0 to 4.2, more preferably 3.5 to 4.5, still more preferably 3.8 to 4.2, yet more preferably 3.9 to 4.1, and particularly preferably 4.0.

In some embodiments, the anti-Nectin4 antibody-drug conjugate or the pharmaceutically acceptable salt thereof in the pharmaceutical combination of the present invention has a molecular formula of Ab-[L-CTD]m, wherein Ab represents an anti-Nectin-4 antibody or an antigen-binding fragment thereof, comprising a heavy chain variable region and a light chain variable region, wherein the heavy chain variable region comprises HCDR1 shown in SEQ ID NO:1,

HCDR2 shown in SEQ ID NO:2, and HCDR3 shown in SEQ ID NO:3; and the light chain variable region comprises LCDR1 shown in SEQ ID NO:4, LCDR2 shown in SEQ ID NO:5, and LCDR3 shown in SEQ ID NO:6;

Preferably, the Ab comprises a heavy chain variable region and a light chain variable region, wherein the heavy chain variable region comprises a sequence shown in SEQ ID NO: 7 or a sequence having at least 90%, 91%, 92%, 93%, 94%, 95%, 96%, 97%, 98%, or 99% identity therewith; and the light chain variable region comprises a sequence shown in SEQ ID NO: 8 or a sequence having at least 90%, 91%, 92%, 93%, 94%, 95%, 96%, 97%, 98%, or 99% identity therewith;

For example, the heavy chain variable region comprises an amino acid sequence shown in SEQ ID NO: 7 or SEQ ID NO: 9, and the light chain variable region comprises an amino acid sequence shown in SEQ ID NO: 8 or SEQ ID NO:10;

More preferably, the Ab comprises:

(i) a heavy chain variable region amino acid sequence shown in SEQ ID NO: 7; and a light chain variable region amino acid sequence shown in SEQ ID NO: 8; or

(ii) a heavy chain variable region amino acid sequence shown in SEQ ID NO: 9; and a light chain variable region amino acid sequence shown in SEQ ID NO:10;

Preferably, the Ab is an IgG antibody, more preferably a human IgG antibody, most preferably a human IgG1 or human IgG4 antibody; and wherein the antigen-binding fragment is Fab, Fab', $F(ab')_2$, Fv, single-chain Fv, or single-chain Fab.

In some embodiments, the anti-Nectin4 antibody-drug conjugate or the pharmaceutically acceptable salt thereof in the pharmaceutical combination of the present invention has a structure shown in Formula Ia and/or Ib below:

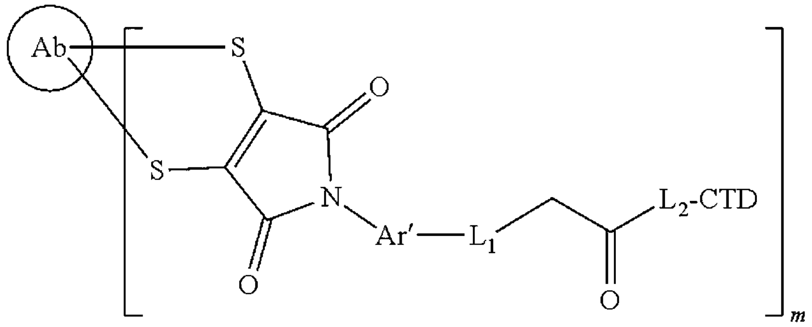

Ia

and/or

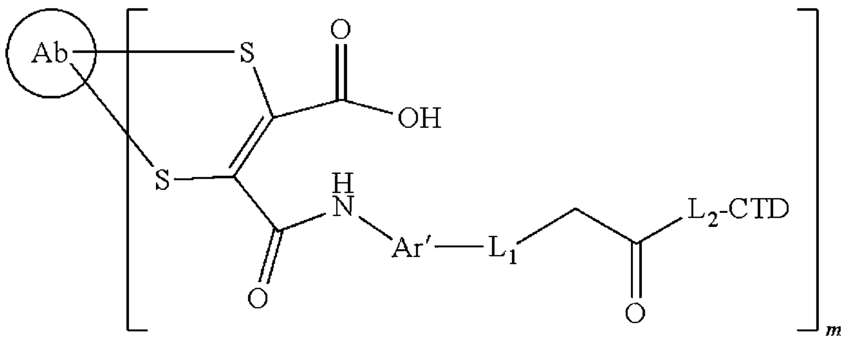

Ib

wherein: Ab is an anti-Nectin-4 antibody or an antigen-binding fragment thereof;

Ar' is any one selected from the group consisting of: substituted or unsubstituted C6-C10 arylene and substituted or unsubstituted 5-12 membered heteroarylene, wherein the substitution refers to the replacement of a hydrogen atom on a group by one or more substituents selected from the group consisting of: halogen (F, Cl, Br or I), halogenated alkyl (e.g. halogenated C1-C6 alkyl, preferably halogenated C1-C4 alkyl, e.g. trifluoromethyl) and alkoxy (e.g. C1-C6 alkoxy, preferably C1-C4 alkoxy, e.g. methoxy);

$L_1$ is -$O(CH_2CH_2O)n$- linked to Ar', wherein n is any integer selected from the group consisting of 1 to 24, preferably any integer from 1 to 10, more preferably any integer from 3 to 5;

$L_2$ is an enzyme cleavable fragment, e.g., a dipeptide or a tripeptide or a tetrapeptide or a combination thereof with a self-immolating structural fragment (i.e., a polypeptide fragment consisting of 2-4 amino acids, or a combination of the polypeptide fragment with a self-immolating structural fragment), such as Val-Ala, Val-Ala-PAB, Val-Cit, Val-Cit-PAB, Phe-Lys-PAB, Ala-Ala-Ala, Gly-Gly-Phe-Gly (GGFG), MAC glucuronide phenol.

In some embodiments, $L_2$-CTD is VcMMAE, GGFG-Dxd, or VC-seco-DUBA. Preferably, if Ar' is a substituted or unsubstituted 5-12 membered heteroarylene, then the heteroatom is N. Preferably, Ar' is a substituted or unsubstituted C6 arylene, or a substituted or unsubstituted 6 membered heteroarylene. More preferably, the antibody-drug conjugate or the pharmaceutically acceptable salt thereof has the following structure:

Conjugate ADC-1:

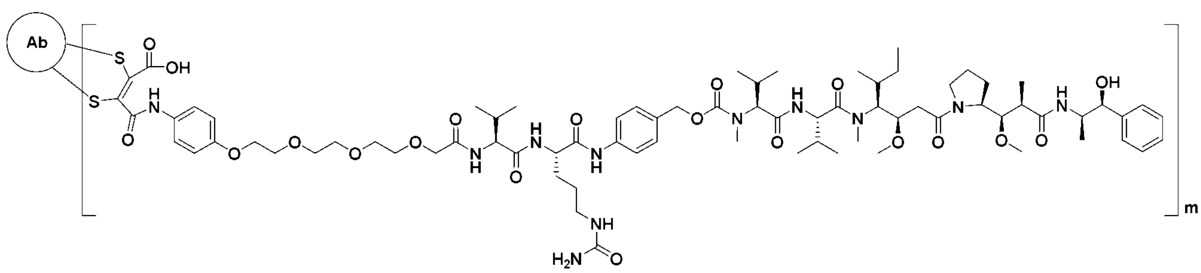

Conjugate ADC-2:

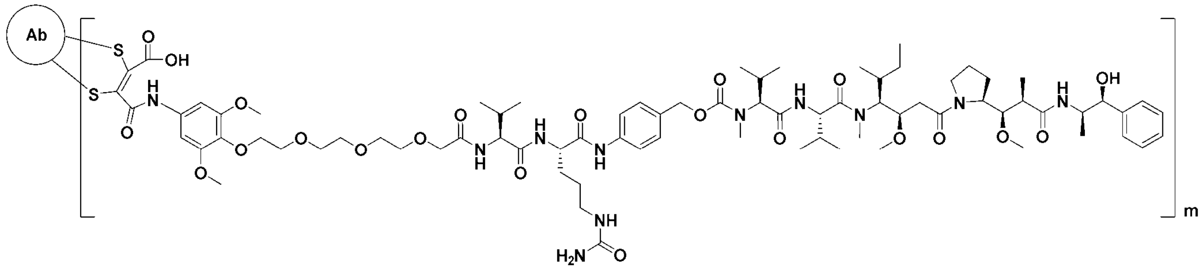

Conjugate ADC-3:

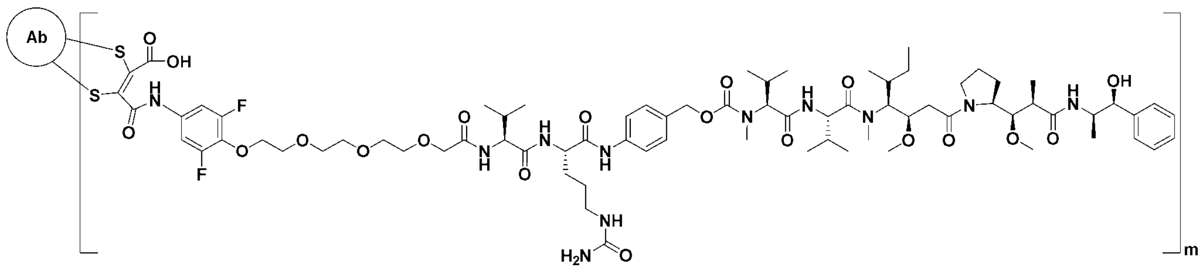

Conjugate ADC-4:

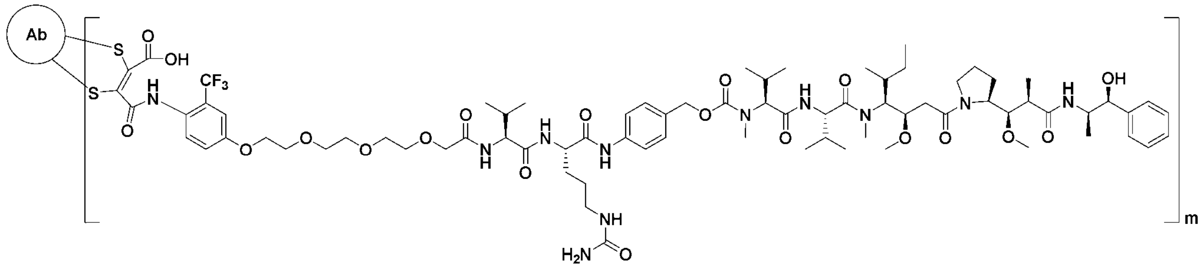

Conjugate ADC-5:

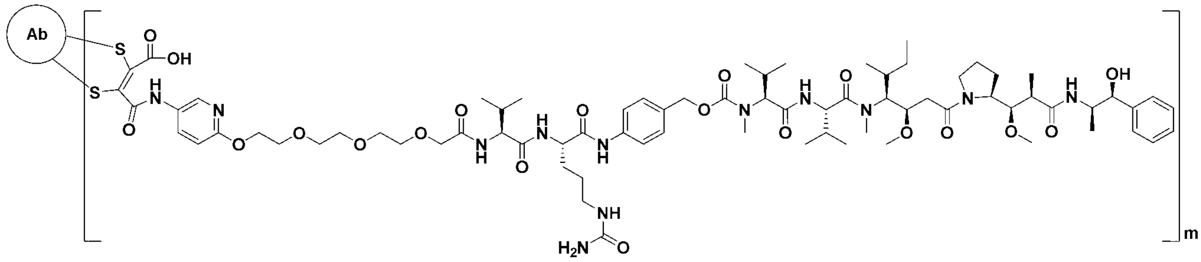

Conjugate ADC-6:

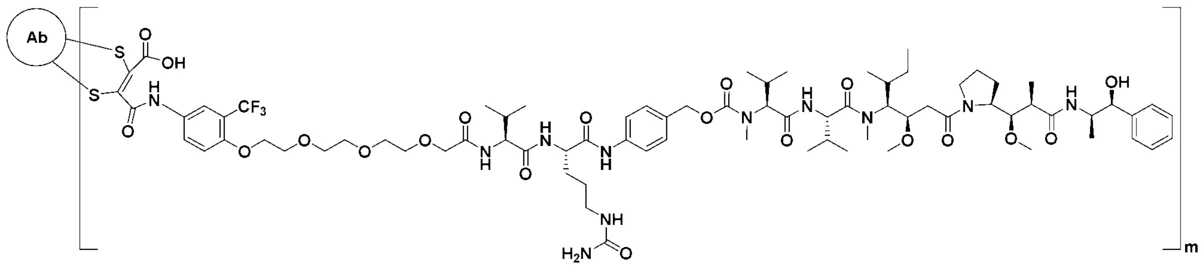

Conjugate ADC-7:

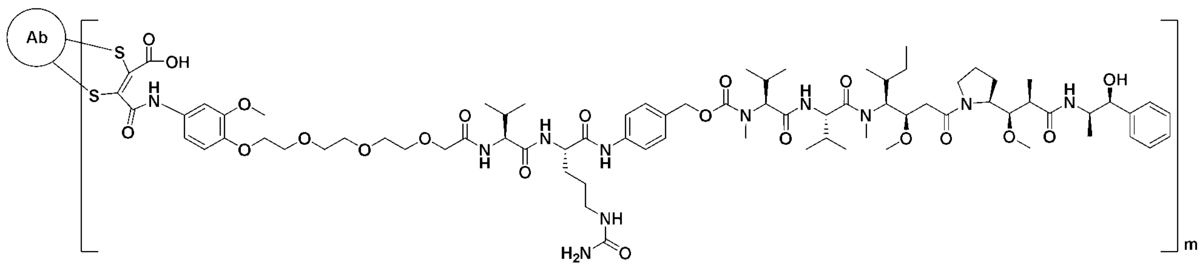

In some embodiments, a pharmaceutical composition of the present invention is provided, wherein

(i) an anti-Nectin4 antibody-drug conjugate or a pharmaceutically acceptable salt thereof is represented by the following formula:

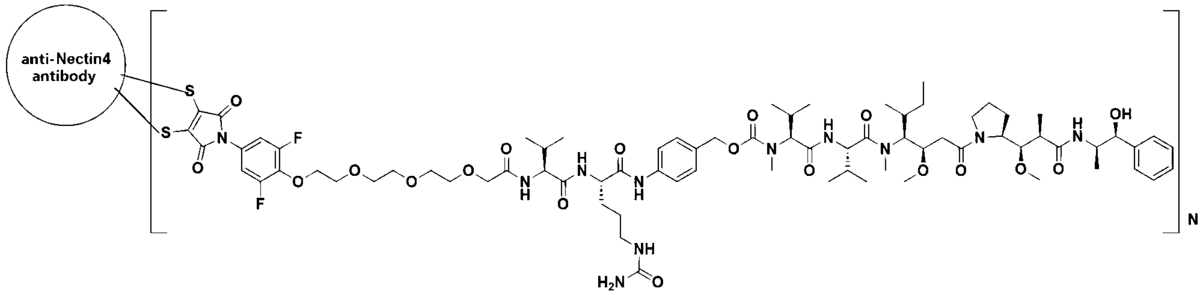

and/or

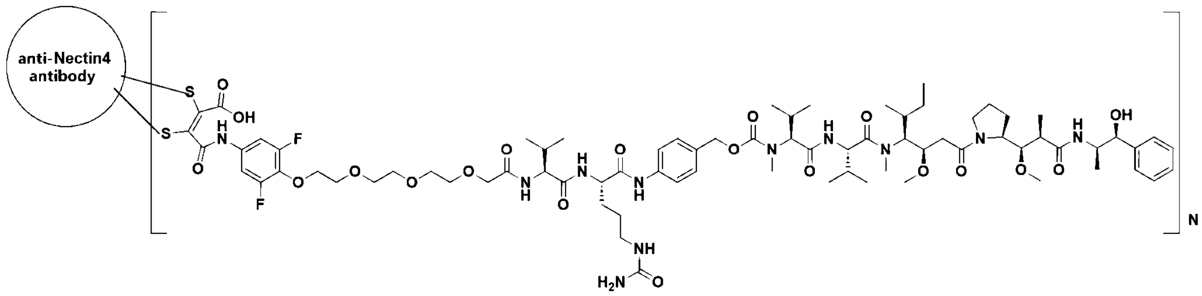

Wherein, the anti-Nectin4 antibody is as defined above,

N represents an average number of drugs conjugated per anti-Nectin-4 antibody molecule, and is 1.0 to 5.0, preferably 3.0 to 4.2, more preferably 3.5 to 4.5, still more preferably 3.8 to 4.2, yet more preferably 3.9 to 4.1, and particularly preferably 4.0; and

(ii) an anti-PD-1 antibody or an antigen-binding fragment thereof is selected from toripalimab or pembrolizumab, or an antigen-binding fragment thereof.

In a second aspect, the present invention provides a use of the pharmaceutical combination described in the first aspect for the manufacture of a medicament for preventing or treating a cancer, preferably the cancer is any one selected from the group consisting of: urothelial carcinoma, bladder cancer, breast cancer, ovarian cancer, pancreatic cancer, hepatocellular cancer, gastric cancer, non-hodgkin's lymphoma, hodgkin's lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, prostate cancer, colorectal cancer, colon cancer, glioma, and mesothelioma.
In a third aspect, the present invention provides a kit of parts comprising the pharmaceutical combination described in the first aspect of the present invention. Preferably, the kit is in a form of a pharmaceutical dosage unit.
Other aspects and embodiments of the present invention will become apparent from the following detailed description.

## Brief Description of the Drawings

The preferred embodiments of the present invention described in detail below will be better understood when read in conjunction with the accompanying drawings. For the purpose of illustrating the present invention, the drawings show currently preferred embodiments. However, it should be understood that the present invention is not limited to the precise arrangement and means of the embodiments shown in the drawings.

Figure 1 shows the effect of the anti-Nectin4-ADC combined with the PD-1 antibody pembrolizumab or administered alone on the tumor volume in tumor-bearing mice.

Figure 2 shows the effect of the anti-Nectin4-ADC combined with the PD-1 antibody pembrolizumab or administered alone on the body weight of tumor-bearing mice.

Figure 3 shows the effect of the anti-Nectin4-ADC combined with the PD-1 antibody toripalimab or administered alone

on the tumor volume in tumor-bearing mice.

Figure 4 shows the effect of the anti-Nectin4-ADC combined with the PD-1 antibody toripalimab or administered alone on the body weight of tumor-bearing mice.

## Detailed Description

Before detailing the present invention, it should be understood that the present invention is not limited to the specific methods and experimental conditions described herein, as the methods and conditions may be modified. Furthermore, the terminology used herein is intended to illustrate specific embodiments and is not intended to be limiting.

## I. Definitions

Unless otherwise specified, all technical and scientific terms used herein shall have the same meanings as commonly understood by a person skilled in the art. For the purpose of the present invention, the following terms are defined.

The term "about" used in conjunction with a numerical value is intended to encompass the numerical values in a range from a lower limit less than the specified numerical value by 10% to an upper limit greater than the specified numerical value by 10%.

The term "and/or" when used to connect two or more options should be understood to mean any one of the options, or any two or more of the options.

As used herein, the term "comprise" or "include" means including the described elements, integers, or steps, but not excluding any other elements, integers, or steps. The term "comprise" or "include" used herein, unless otherwise specified, also encompasses the scenario where the subject matter consists of the described elements, integers or steps. For example, when referring to an antibody variable region "comprising" a particular sequence, it is also intended to encompass an antibody variable region consisting of the particular sequence.

The term "antibody" is used in its broadest sense to refer to a protein comprising an antigen-binding site, encompassing natural and artificial antibodies of various structures, including but not limited to whole antibodies and antigen-binding fragments of antibodies.

The terms "whole antibody", "full-length antibody", "complete antibody", and "intact antibody" are used interchangeably herein to refer to a glycoprotein comprising at least two heavy chains (H) and two light chains (L) interconnected by disulfide bonds. Each heavy chain consists of a heavy chain variable region (abbreviated as VH herein) and a heavy chain constant region. The heavy chain constant region consists of three domains: CH1, CH2, and CH3. Each light chain consists of a light chain variable region (abbreviated as VL herein) and a light chain constant region. The light chain constant region consists of a domain, CL. The VH and VL regions can be further subdivided into hypervariable regions (complementarity-determining regions, CDRs), interspersed with more conserved regions (framework regions, FRs). Each VH and VL comprises three CDRs and four FRs, arranged in the following order from amino terminus to carboxyl terminus: FR1, CDR1, FR2, CDR2, FR3, CDR3, FR4. The constant regions do not directly participate in antibody-antigen binding but exhibit multiple effector functions. Within a given VH or VL amino acid sequence, the precise amino acid sequence boundaries of each CDR can be determined using any one or combination of several well-known schemes, including for example: the Chothia numbering scheme (Chothia et al., "Canonical structures for the hypervariable regions of immunoglobulins", Journal of Molecular Biology, 196, 901-917 (1987)); the Kabat numbering scheme (Kabat et al., "Sequences of Proteins of Immunological Interest, 4th ed., U.S. Department of Health and Human Services, National Institutes of Health (1987)), AbM (University of Bath), and Contact (University College London); the North numbering scheme (North et al., "A New Clustering of Antibody CDR Loop Conformations", Journal of Molecular Biology, 406, 228-256 (2011)). The boundaries of the CDRs of the anti-Nectin4 antibody in the anti-Nectin4 antibody-drug conjugate of the present invention and the boundaries of the CDRs of the anti-PD-1 antibody used in combination with the anti-Nectin4 antibody-drug conjugate may be determined according to any scheme in the art or a combination thereof, and may be determined by artificial assessment. In one embodiment, the CDRs of the antibodies of the present invention are defined as CDR sequences according to the Kabat numbering scheme.

Although CDRs differ between antibodies, only a limited number of amino acid positions within CDRs directly participate in antigen binding. By employing at least two of the Kabat, Chothia, AbM, and Contact methods, a minimal overlapping region can be identified, thereby providing the "minimal binding unit" for antigen binding. The minimal binding unit can constitute a subset of a CDR. As those skilled in the art will appreciate, the remaining residues in the CDR sequence can be determined based on the antibody's structure and protein folding. Therefore, the present invention also contemplates any variants of the CDRs disclosed herein. For example, in a variant of a CDR, the amino acid residues of the minimal binding unit may remain unchanged, while the remaining residues in the CDR, as defined according to Kabat or Chothia, may be substituted with conserved amino acid residues.

The term "antigen-binding fragment" refers to a portion or segment of a complete or full-length antibody, comprising fewer amino acid residues than the complete or full-length antibody, and capable of binding an antigen or competing with the complete antibody (i.e., the complete antibody from which the antigen-binding fragment is derived) for binding to the antigen. Antigen-binding fragments may be prepared by recombinant DNA technology, or by enzymatic or chemical cleavage of complete antibodies. Antigen-binding fragments include, but are not limited to, Fab, Fab', $F(ab')_2$, Fv, single-chain Fv, diabody, and single-domain antibody (sdAb). The Fab fragment is a monovalent fragment consisting of VL, VH, CL, and CH1 domains, and is obtainable, e.g., by papain digestion of a whole antibody. Additionally, $F(ab')_2$, a dimer of Fab', is generated by digestion of a complete antibody with pepsin below the disulfide bond in the hinge region. $F(ab')_2$ is a bivalent antibody fragment. $F(ab')_2$ can be reduced under neutral conditions by breaking the disulfide bonds in the hinge region, thereby converting the $F(ab')_2$ dimer into Fab' monomers. The Fab' monomer is essentially a Fab fragment with a hinge region (for more detailed descriptions of other antibody fragments, see: Fundamental Immunology, edited by W. E. Paul, Raven Press, N.Y. (1993)). The Fv fragment consists of VL and VH domains of an antibody single arm. Additionally, although the two domains VL and VH of the Fv fragment are encoded by separate genes, a recombinant method can link them via a synthetic linker peptide that allows the domains to be expressed as a single protein chain. Within this single protein chain, the VL and VH regions pair to form a single-chain Fv. The antibody fragments can be obtained chemically, by recombinant DNA methods, or through protease digestion.

The term "monoclonal antibody" refers to peptides with essentially identical amino acid sequences or derived from the same genetic source, including antibodies and antigen-binding fragments. The term also encompasses formulations of an antibody molecule consisting of a single molecule. Monoclonal antibodies exhibit unique binding specificity and affinity for specific epitopes.

The term "humanized" antibody refers to a chimeric antibody comprising amino acid residues from non-human CDRs and amino acid residues from human FRs. In some embodiments, a humanized antibody comprises all or substantially all CDRs corresponding to those in a non-human antibody and all or substantially all FR regions corresponding to those in a human antibody. A humanized antibody may optionally comprise at least a portion of antibody constant region derived from a human antibody. The "humanized form" of an antibody (e.g., a non-human antibody) refers to an antibody that has undergone humanization.

The term "specific binding" and the like refer to the formation of a complex between an antibody and an antigen that is relatively stable under physiological conditions. Methods for determining whether an antibody specifically binds to an antigen are well known in the art and include, for example, surface plasmon resonance assay, MSD assay (Estep, P. et al., High throughput solution-based measurement of antibody-antigen affinity and epitope binning, MAbs, 2013. 5(2): p. 270-278), and the like.

In the context of two or more nucleic acid sequences or polypeptide sequences, the term "% identity" refers to the identity percentage between sequences. Two sequences are "identical" over the compared region if they have the same amino acid sequence or nucleotide sequence. Two sequences are "substantially identical" if, when aligned and compared over a comparison window or designated region to achieve maximum correspondence as measured using one of the following sequence comparison algorithms or by manual alignment and visual inspection, they share a specified percentage of identical amino acid residues or nucleotides (i.e., 60% identity, optionally 65%, 70%, 75%, 80%, 85%, 90%, 95%, or 99% identity over a designated region, or over the entire sequence if no region is designated). The calculation of sequence identity between sequences is performed as follows.

To determine the percent identity of two amino acid sequences or two nucleic acid sequences, the sequences are aligned for optimal comparison purposes (for example, gaps can be introduced in either or both of the first and second amino acid sequences or nucleic acid sequences for optimal alignment or non-homologous sequences can be discarded for comparison purposes). In a preferred embodiment, the length of a reference sequence aligned for comparison purposes is at least 30%, preferably at least 40%, more preferably at least 50%, 60% and even more preferably at least 70%, 80%, 90% and 100% of the length of the reference sequence. The amino acid residues or nucleotides at corresponding amino acid positions or nucleotide positions are then compared. When the position in the first sequence is occupied by the same amino acid residue or nucleotide as that at the corresponding position in the second sequence, the molecules are identical at that position.

The sequence comparison and the calculation of percent identity between two sequences can be achieved using a mathematical algorithm. In a preferred embodiment, the percent identity between two amino acid sequences is determined using the Needlema-Wunsch ((1970) J. Mol. Biol. 48: 444-453) algorithm which has been incorporated into a GAP program in a GCG software package (available at http://www.gcg.com), and using a Blossum 62 matrix or a PAM250 matrix, a gap weight of 16, 14, 12, 10, 8, 6 or 4 and a length weight of 1, 2, 3, 4, 5 or 6. In another preferred embodiment, the percent identity between two nucleotide sequences is determined using the GAP program in the GCG software package (available at http://www.gcg.com) and using an NWSgapdna.CMP matrix, a gap weight of 40, 50, 60, 70 or 80 and a length weight of 1, 2, 3, 4, 5 or 6. A particularly preferred parameter set (and one parameter set that should be used unless otherwise stated) is a Blossum 62 scoring matrix with a gap penalty of 12, a gap extension penalty of 4 and a gap frameshift penalty of 5.

The percent identity between two amino acid sequences or nucleotide sequences can also be determined using a PAM120 weighted remainder table, with a gap length penalty of 12 and a gap penalty of 4, and using the E. Meyers and W. Miller algorithm ((1989) CABIOS, 4:11-17) that has been incorporated into the ALIGN program (version 2.0).

Additionally or alternatively, the nucleic acid sequences and protein sequences described herein can further be used as "query sequences" to perform searches against public databases, e.g., to identify other family member sequences or related sequences.

The term "immune checkpoint" refers to a class of inhibitory signaling molecules present in the immune system. These molecules regulate the duration and intensity of immune responses in peripheral tissues to prevent tissue damage, and are involved in maintaining tolerance to self-antigens (Pardoll DM., The blockade of immune checkpoints in cancer immunotherapy. Nat Rev Cancer, 2012, 12(4): 252-264). Research has found that one of the reasons why tumor cells can evade the body's immune system and proliferate uncontrollably is their exploitation of the inhibitory signaling pathways of immune checkpoints, thereby suppressing the activity of T lymphocytes and preventing T lymphocytes from effectively exerting tumor-killing effects (Yao S, Zhu Y and Chen L., Advances in targeting cell surface signaling molecules for immune modulation. Nat Rev Drug Discov, 2013, 12(2):130-146). Immune checkpoint molecules include, but are not limited to, programmed death-1 (PD-1), PD-L1, PD-L2, cytotoxic T-lymphocyte-associated antigen 4 (CTLA-4), LAG-3, TIM-3, and a protein with T cell immunoglobulin and ITIM domains (TIGIT).

The term "pharmaceutical combination" refers to a non-fixed combination product or a fixed combination product, including but not limited to a kit of parts and a pharmaceutical composition. The term "non-fixed combination" means that the active ingredients (e.g., (i) the anti-Nectin4 antibody-drug conjugate or pharmaceutically acceptable salt thereof, and (ii) the anti-PD-1 antibody or antigen-binding fragment thereof) are administered to a patient as separate entities simultaneously, without a specific time limit, or sequentially at the same or different intervals, wherein such administration provides prophylactically or therapeutically effective amounts of the two active agents in the patient. In some embodiments, the anti-PD-1 antibody or antigen-binding fragment thereof and the anti-Nectin4 antibody-drug conjugate or pharmaceutically acceptable salt thereof used in the pharmaceutical combination are administered at amounts not exceeding those when they are used alone. The term "fixed combination" means the two active agents are administered to a patient simultaneously as a single entity. The dosages and/or timing intervals for the two active agents are preferably selected such that the combined use of each component produces a greater therapeutic effect in treating a disease or disorder than achievable by using either component alone. The components may each be in separate dosage forms, and the dosage forms may be the same or different.

The terms "administration" and "dosing" are used interchangeably and refer to the physical introduction of each of the active ingredients in the pharmaceutical combination of the present invention into an individual by any of a variety of methods and delivery systems known to those skilled in the art. The routes of administration of each active ingredient in the pharmaceutical combination of the present invention include oral, intravenous (e.g., infusion (also referred to as intravenous drip) or injection), intramuscular, subcutaneous, intraperitoneal, spinal, local, or other parenteral administration routes. The term "parenteral administration" refers to administration other than gastrointestinal or local routes, typically via intravenous route, and includes, without limitation, intramuscular, intra-arterial, intrathecal, intralymphatic, intralesional, intra-cystic, intra-orbital, intracardiac, intradermal, intraperitoneal, transtracheal, subcutaneous, subepidermal, intra-articular, sub-cystic, subarachnoid, intraspinal, epidural, and intrasternal injections and infusions, as well as in vivo electroporation. Accordingly, each of the active ingredients in the pharmaceutical combination of the present invention may be formulated as capsules, tablets, injections (including infusion solutions or injection solutions), syrups, sprays, lozenges, liposomes, or suppositories, and the like.

The term "dose" refers to an amount of a drug that elicits a therapeutic effect. Unless otherwise specified, a dose relates to the amount of the drug in its free form. If the drug is in the form of a pharmaceutically acceptable salt, the amount of the drug is increased proportionally compared with the amount of the drug in its free form. For example, the dose will be recited on the product packaging or in the product information leaflet.

The term "pharmaceutically acceptable" refers to compounds, materials, compositions, and/or dosage forms suitable for use in contact with human and animal tissues without excessive toxicity, irritation, allergic reactions, or other problems or complications, and commensurate with a reasonable benefit/risk ratio.

The term "cancer" refers to a disease characterized by the proliferation of abnormal cells with rapid and uncontrolled growth. Cancer cells may be locally or spread to other parts of the body via the bloodstream and lymphatic system. Cancers include, but are not limited to, solid tumors and hematological malignancies, preferably solid tumors. Examples of various cancers include but are not limited to urothelial carcinoma, bladder cancer, breast cancer, ovarian cancer, pancreatic cancer, hepatocellular cancer, gastric cancer, non-hodgkin's lymphoma, hodgkin's lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, prostate cancer, colorectal cancer, colon cancer, glioma, and mesothelioma. The cancers are preferably advanced cancers, recurrent and/or refractory cancers, or cancers resistant to chemotherapy, more preferably advanced solid tumors, such as (histologically or cytologically confirmed) unresectable or metastatic advanced solid tumors.

The term "inhibition" refers to a reduction in certain parameters (e.g., Nectin-4 activity and/or PD-1 activity) resulted from a given molecule (e.g., (i) the anti-Nectin4 antibody-drug conjugate or pharmaceutically acceptable salt thereof, and/or (ii) the anti-PD-1 antibody or antigen-binding fragment thereof). For example, the term encompasses inhibition of at least 5%, 10%, 20%, 30%, 40%, or more of activity. Thus, inhibition need not be 100%.

The term "treatment" encompasses the administration of the pharmaceutical combination of the present invention to individuals in need thereof, for the purpose of curing a disease or achieving regression of the disease, or delaying its progression. When referring to a disease, the term "treatment" means alleviating the disease (i.e., slowing or inhibiting or reducing the progression of said disease or at least one of its clinical symptoms), preventing or delaying the onset or development or progression of the disease.

The term "prevention" encompasses the suppression or delay of the occurrence or frequency of a disease, a disorder, or its symptoms. It typically refers to the administration of a drug prior to the onset of signs or symptoms, particularly in individuals at risk.

The term "individual" refers to mammals and non-mammals. Mammals refer to any member of the class Mammalia, including but not limited to: humans; non-human primates; cattle, horses, sheep, pigs, rabbits, dogs, and cats, etc. The term "individual" does not limit a specific age or gender. In some embodiments, the individual is a human.

The term "adverse event" (AE) refers to any unfavorable and typically unintended or undesirable sign (including abnormal laboratory findings), symptom, or disease associated with the use of a medical treatment. For example, an adverse event may relate to the activation of the immune system in response to treatment or the expansion of immune system cells (e.g., T cells) in response to treatment. A medical treatment may be associated with one or more AEs, and each AE may have the same or different levels of severity.

The term "overall survival" or "OS" refers to the time from a patient's first use of the study drug to his death from any cause.

The term "progression-free survival" or "PFS" refers to the time from a patient's first use of the study drug to the occurrence of disease progression or death from any cause.

All numerical ranges disclosed herein shall be understood to encompass every value and subset of values within the range, regardless of whether they are specifically disclosed. For example, reference to any numerical range shall be deemed to include every value within that range, such as every integer within the numerical range. The present invention encompasses all values falling within these ranges, all smaller ranges, and the upper or lower bounds of the numerical ranges.

Technical and scientific terms used herein that are not specifically defined shall have the meanings commonly understood by those skilled in the art to which the present invention pertains.

**II. Pharmaceutical combination of the present invention**

The pharmaceutical combination of the present invention comprises (i) an anti-Nectin4 antibody-drug conjugate or a pharmaceutically acceptable salt thereof; and (ii) an anti-PD-1 antibody or an antigen-binding fragment thereof.

(i) Anti-Nectin4 antibody-drug conjugate or pharmaceutically acceptable salt thereof

An antibody-drug conjugate (ADC) is a conjugate that employs a technology that leverages the ability of an antibody's specific recognition of a particular antigen on a tumor cell surface, to precisely deliver anti-tumor drugs (such as cytotoxic agents, cell inhibitors, small-molecule chemotherapeutics, etc.) to target tumor cells, and intracellularly accumulate and release, thereby achieving precise tumor cell killing. ADC typically consists of three components: an antibody or antibody-like ligand, a small-molecule drug, and a linker (connector) that conjugates the antibody or antibody-like ligand to the drug. Due to its appropriate molecular weight, high stability, strong targeting capability, and reduced toxicity and side effects, an antibody-drug conjugate is recognized as the most promising anti-tumor drug.

The anti-Nectin4 antibody-drug conjugate in the pharmaceutical combination of the present invention consists of three components: an antibody or an antigen-binding fragment thereof that specifically binds to Nectin-4, a small-molecule drug, and a linker (connector) that conjugates the antibody to the small-molecule drug.

In some embodiments, the anti-Nectin4 antibody-drug conjugate has a molecular formula of Ab-[L-CTD]m, wherein

Ab represents an anti-Nectin-4 antibody or an antigen-binding fragment thereof,

L represents a linker,

CTD represents a drug, and

m represents an average number of drugs conjugated per Ab molecule.

In some embodiments, the Ab in the molecular formula of Ab-[L-CTD]m comprises a heavy chain variable region and a light chain variable region, wherein the heavy chain variable region comprises HCDR1 shown in SEQ ID NO:1, HCDR2 shown in SEQ ID NO:2, and HCDR3 shown in SEQ ID NO:3; and the light chain variable region comprises LCDR1 shown in SEQ ID NO:4, LCDR2 shown in SEQ ID NO:5, and LCDR3 shown in SEQ ID NO:6;

Preferably, the Ab comprises a heavy chain variable region and a light chain variable region, wherein the heavy chain variable region comprises a sequence shown in SEQ ID NO: 7 or a sequence having at least 90%, 91%, 92%, 93%, 94%, 95%, 96%, 97%, 98%, or 99% identity therewith, and the light chain variable region comprises a sequence shown in SEQ ID NO: 8 or a sequence having at least 90%, 91%, 92%, 93%, 94%, 95%, 96%, 97%, 98%, or 99% identity therewith;

For example, the heavy chain variable region comprises an amino acid sequence shown in SEQ ID NO: 7 or SEQ ID NO: 9, and the light chain variable region comprises an amino acid sequence shown in SEQ ID NO: 8 or SEQ ID NO:10;

More preferably, the Ab comprises:

- a heavy chain variable region amino acid sequence shown in SEQ ID NO: 7; and a light chain variable region amino acid sequence shown in SEQ ID NO: 8; or
- a heavy chain variable region amino acid sequence shown in SEQ ID NO: 9; and a light chain variable region amino acid sequence shown in SEQ ID NO:10;

Preferably, the Ab is an IgG antibody; more preferably, a human IgG antibody; most preferably, a human IgG1 or human IgG4 antibody; wherein the antigen-binding fragment is Fab, Fab', $F(ab')_2$, Fv, single-chain Fv, or single-chain Fab.

In some embodiments, L in the molecular formula of Ab-[L-CTD]m is any chemical moiety capable of covalently linking the CTD to the Ab. A crosslinking reagent is a bifunctional or multifunctional reagent that can be used to link the CTD and the Ab to form an antibody-drug conjugate. An antibody-drug conjugate can be prepared using a crosslinking reagent with reactive functional groups capable of binding to both the CTD and the Ab. For example, cysteine, thiol, or amine, e.g., at the N-terminus of the antibody or at the side chain of an amino acid like lysine, can form a bond with the functional group of the crosslinking reagent. Alternatively, the antibody-drug conjugate may be prepared by preforming a linker-payload (i.e., a linker-drug conjugate) and reacting the linker-payload with the antibody. In one embodiment, L is a cleavable linker. In another embodiment, L is a non-cleavable linker. In some embodiments, L is an acid-labile linker, a photo-labile linker, a peptidase cleavable linker, an esterase cleavable linker, a disulfide bond cleavable linker, a hydrophilic linker, a precharged linker, a glycosidase-cleavable linker, a phosphodiesterase-cleavable linker, a phosphatase-cleavable linker, or a dicarboxylic acid-based linker.

In some embodiments, the CTD in the molecular formula of Ab-[L-CTD]m is a cytotoxic drug. In preferred embodiments, the CTD is selected from one or more of the following: microtubule inhibitors MMAE, DM1, DM4, Tublysin, amanitin, calicheamicin, Eribulin and derivatives thereof; topoisomerase inhibitors SN38, Exatecan and derivatives thereof; and DNA binding agents PBD, doxorubicin and derivatives thereof. The value of m is 1.0 to 5.0, preferably 3.0 to 4.2, more preferably 3.5 to 4.5, still more preferably 3.8 to 4.2, yet more preferably 3.9 to 4.1, and particularly preferably 4.0.

In some embodiments, the anti-Nectin-4 antibody-drug conjugate or the pharmaceutically acceptable salt thereof has a structure shown in Formula Ia and/or Ib below:

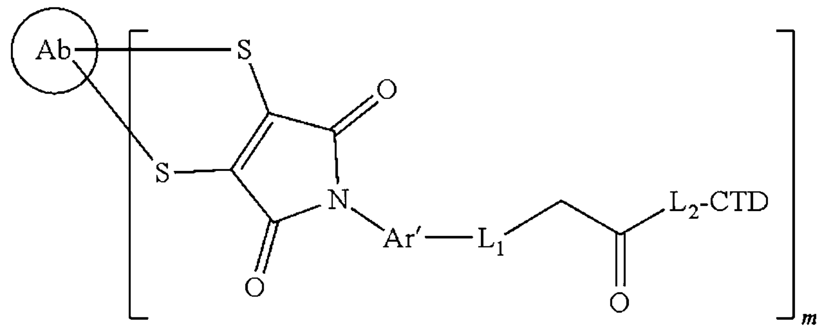

Ia

and/or

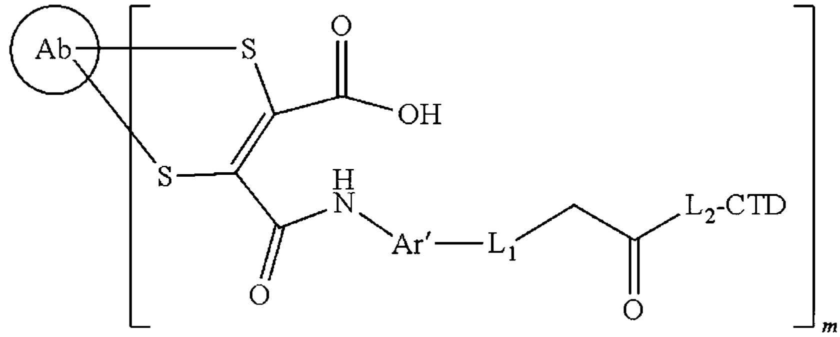

Ib

wherein: Ab is the anti-Nectin-4 antibody or the antigen-binding fragment thereof as defined above;

Ar' is any one selected from the group consisting of: substituted or unsubstituted C6-C10 arylene and substituted or unsubstituted 5-12 membered heteroarylene, wherein the substitution refers to the replacement of a hydrogen atom on a group by one or more substituents selected from the group consisting of: halogen (F, Cl, Br or I), halogenated alkyl (e.g. halogenated C1-C6 alkyl, preferably halogenated C1-C4 alkyl, e.g. trifluoromethyl) and alkoxy (e.g. C1-C6 alkoxy, preferably C1-C4 alkoxy, e.g. methoxy);

$L_1$ is $-O(CH_2CH_2O)n-$ linked to Ar', wherein n is any integer selected from the group consisting of 1 to 24, preferably any integer from 1 to 10, more preferably any integer from 3 to 5;

$L_2$ is an enzyme cleavable fragment, e.g., a dipeptide or a tripeptide or a tetrapeptide or a combination thereof with a self-immolating structural fragment (i.e., a polypeptide fragment consisting of 2-4 amino acids, or a combination of the polypeptide fragment with a self-immolating structural fragment), such as Val-Ala, Val-Ala-PAB, Val-Cit, Val-Cit-PAB, Phe-Lys-PAB, Ala-Ala-Ala, Gly-Gly-Phe-Gly (GGFG), MAC glucuronide phenol.

In some embodiments, $L_2$-CTD is VcMMAE, GGFG-Dxd, or VC-seco-DUBA. Preferably, if Ar' is a substituted or unsubstituted 5-12 membered heteroarylene, then the heteroatom is N. Preferably, Ar' is a substituted or unsubstituted C6 arylene, or a substituted or unsubstituted 6 membered heteroarylene. More preferably, the antibody-drug conjugate or the pharmaceutically acceptable salt thereof has the structure of conjugate ADC-1, conjugate ADC-2, conjugate ADC-3, conjugate ADC-4, conjugate ADC-5, conjugate ADC-6, or conjugate ADC-7 described above.

In some embodiments, the anti-Nectin-4 antibody-drug conjugate or the pharmaceutically acceptable salt thereof is represented by the following formula:

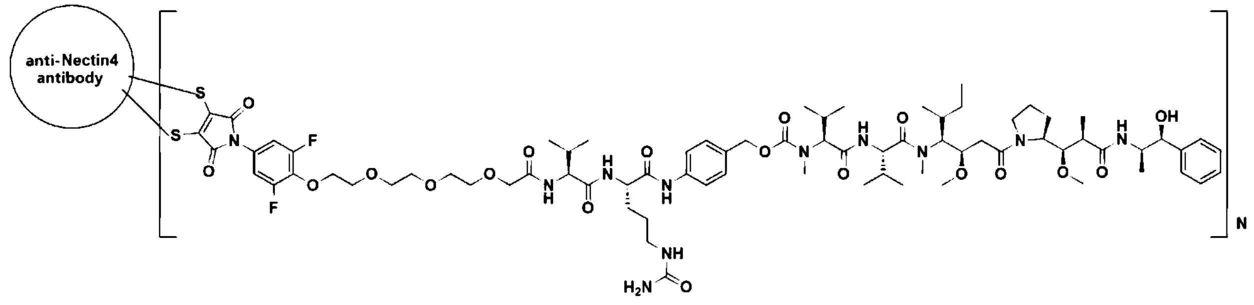

and/or

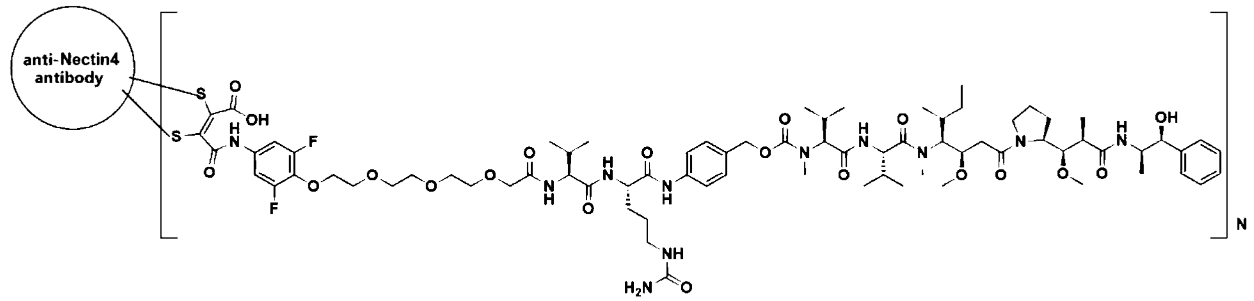

Wherein, the anti-Nectin4 antibody is as defined above,

N represents an average number of drugs conjugated per anti-Nectin-4 antibody molecule, and is 1.0 to 5.0, preferably 3.0 to 4.2, more preferably 3.5 to 4.5, still more preferably 3.8 to 4.2, yet more preferably 3.9 to 4.1, and particularly preferably 4.0;

In another embodiment, the anti-Nectin4 antibody-drug conjugate is PADCEV™ (also known as enfortumab vedotin-ejfv; Chinese name: enfortumab vedotin), which targets the Nectin-4 protein highly expressed in urothelial carcinoma. It is FDA-approved for the treatment of locally advanced or metastatic urothelial carcinoma. This ADC drug, which is formed by conjugating enfortumab, a human IgG1 monoclonal antibody targeting the Nectin-4 protein, with a cytotoxic agent MMAE (monomethyl auristatin E, a microtubule disruptor) (Pia M. Challita-Eid et al. (2016) Cancer Res.76(10):3003-13). However, PADCEV™ may cause severe skin adverse reactions, including Stevens-Johnson syndrome (SJS) and toxic epidermal necrolysis (TEN) (2019 FDA announcement).

(ii) Anti-PD-1 antibody or antigen-binding fragment thereof

The anti-PD-1 antibody or antigen-binding fragment thereof in the pharmaceutical combination of the present invention binds to PD-1 expressed on the surface of T lymphocytes, thereby blocking PD-1 binding to its ligand. This promotes T lymphocyte activation, proliferation, and production of immune-activating cytokines such as IL-2, thereby exerting an anti-tumor effect.

In one embodiment, the anti-PD-1 antibody in the pharmaceutical combination of the present invention comprises six CDRs, wherein HCDR1, HCDR2, and HCDR3 are composed of the amino acid sequences shown in SEQ ID NO:11, SEQ ID NO:12, and SEQ ID NO:13, respectively, and wherein LCDR1, LCDR2, and LCDR3 are composed of the amino acid sequences shown in SEQ ID NO:14, SEQ ID NO:15, and SEQ ID NO:16, respectively. The CDR sequences are defined according to the Kabat numbering scheme.

Preferably, the anti-PD-1 antibody comprises a heavy chain variable region VH and a light chain variable region VL, wherein the heavy chain variable region comprises a sequence of SEQ ID NO: 17 or a sequence having at least 90%, 95%, 98%, or 99% identity therewith, and the light chain variable region comprises a sequence of SEQ ID NO:18 or a sequence having at least 90%, 95%, 98%, or 99% identity therewith.

Preferably, the anti-PD-1 antibody comprises a heavy chain sequence of SEQ ID NO:19 or a sequence having at least 90%, 95%, 98%, or 99% identity therewith, and a light chain sequence of SEQ ID NO:20 or a sequence having at least 90%, 95%, 98%, or 99% identity therewith.

Preferably, the anti-PD-1 antibody is toripalimab.

In another embodiment, the anti-PD-1 antibody in the pharmaceutical combination of the present invention comprises six CDRs, wherein HCDR1, HCDR2, and HCDR3 are composed of the amino acid sequences shown in SEQ ID NO:21, SEQ ID NO:22, and SEQ ID NO:23, respectively, and wherein LCDR1, LCDR2, and LCDR3 are composed of the

amino acid sequences shown in SEQ ID NO:24, SEQ ID NO:25, and SEQ ID NO:26, respectively. The CDR sequences are defined according to the Kabat numbering scheme.

Preferably, the anti-PD-1 antibody comprises a heavy chain variable region VH and a light chain variable region VL, wherein the heavy chain variable region comprises a sequence of SEQ ID NO: 27 or a sequence having at least 90%, 95%, 98%, or 99% identity therewith, and the light chain variable region comprises a sequence of SEQ ID NO:28 or a sequence having at least 90%, 95%, 98%, or 99% identity therewith.

Preferably, the anti-PD-1 antibody comprises a heavy chain sequence of SEQ ID NO:29 or a sequence having at least 90%, 95%, 98%, or 99% identity therewith, and a light chain sequence of SEQ ID NO:30 or a sequence having at least 90%, 95%, 98%, or 99% identity therewith.

Preferably, the anti-PD-1 antibody is pembrolizumab.

Other anti-PD-1 antibodies contemplated for use in the pharmaceutical combination of the present invention include MEDI0680 (US Patent No. 8609089), BGB-A317 (US Patent Publication No. 2015/0079109), INCSHR1210 (SHR-1210) (WO2015/085847), REGN-2810 (WO2015/112800), PDR001 (WO2015/112900), TSR-042 (ANB011) (WO2014/179664), and STI-1110 (WO2014/194302).

**III. Use of the pharmaceutical combination of the present invention and methods of treatment using the pharmaceutical combination of the present invention**

The present invention provides the pharmaceutical combination described above for preventing and/or treating the severity of at least one symptom or indication of cancer in an individual, or for suppressing the growth of cancer cells.

The present invention provides a method for preventing or treating a cancer, comprising administering an effective amount of the pharmaceutical combination of the present invention to an individual in need thereof. The effective amount includes a prophylactic effective amount and a therapeutic effective amount.

The present invention provides a use of the aforementioned pharmaceutical combination of the present invention for the manufacture of a medicament for preventing or treating a cancer.

The cancers described herein include solid tumors and hematological malignancies, e.g., urothelial carcinoma, bladder cancer, breast cancer, ovarian cancer, pancreatic cancer, hepatocellular cancer, gastric cancer, non-hodgkin's lymphoma, hodgkin's lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, prostate cancer, colorectal cancer, colon cancer, glioma, and mesothelioma. The cancers are preferably advanced cancers, refractory cancers, and/or cancers resistant to chemotherapy, more preferably advanced solid tumors, (histologically or cytologically confirmed) unresectable or metastatic advanced solid tumors. The cancers are preferably intestinal cancers (including colorectal cancer, colon cancer) and lung cancers (including non-small cell lung cancer). The cancers are preferably advanced recurrent or metastatic cancers (advanced malignant tumors).

The pharmaceutical combination of the present invention may be administered to individuals who have been treated with one or more previous therapies but subsequently experienced recurrence or metastasis.

The pharmaceutical combination of the present invention is administered to individuals exhibiting elevated levels of one or more cancer-associated biomarkers [e.g., Nectin-4, programmed death-ligand 1 (PD-L1)]. For example, a prophylactic or therapeutic effective amount of the pharmaceutical combination of the present invention is administered to individuals with elevated Nectin-4 levels and/or elevated PD-L1 levels.

The anti-Nectin4 antibody-drug conjugate or pharmaceutically acceptable salt thereof in the pharmaceutical combination of the present invention may be administered to individuals in need thereof in one or more doses. For example, in the case of multiple doses, the next dose may be administered 1, 2, 3, 4, 5, 6, 7, 8, 9, or 10 weeks after the previous dose.

The anti-PD-1 antibody or antigen-binding fragment thereof in the pharmaceutical combination of the present invention may be administered to individuals in need thereof in one or more doses. For example, in the case of multiple doses, the next dose may be administered 1,2, 3, 4, 5, 6, 7, 8, 9, or 10 weeks after the previous dose.

The pharmaceutical combination of the present invention may be in any dosage form known to those skilled in the art, such as tablets, capsules, granules, syrups, powders, lozenges, sachets, cachets, elixirs, suspensions, emulsions, solutions, syrups, aerosols, ointments, creams, and injections, etc. Among them, the anti-Nectin4 antibody-drug conjugate or pharmaceutically acceptable salt thereof, and the anti-PD-1 antibody or antigen-binding fragment thereof may each be in a separate dosage form, wherein the dosage forms may be different or the same. The anti-Nectin4 antibody-drug conjugate or pharmaceutically acceptable salt thereof may be in a formulation for intravenous administration or an injection for intraperitoneal administration. The anti-PD-1 antibody or antigen-binding fragment thereof may be in a formulation for intravenous administration or an injection for intraperitoneal administration.

The pharmaceutical combination of the present invention may be in a form of a pharmaceutical dosage unit, such as a single dosage unit.

The anti-Nectin4 antibody-drug conjugate or pharmaceutically acceptable salt thereof and the anti-PD-1 antibody or antigen-binding fragment thereof in the pharmaceutical combination of the present invention may be administered separately, simultaneously, or sequentially.

In some embodiments, administration of at least one cycle of the pharmaceutical combination of the present invention results in an increase, preferably a synergistic increase, in a patient's progression-free survival (PFS) or overall survival (OS) compared with patients administered monotherapy with the anti-Nectin4 antibody-drug conjugate or pharmaceutically acceptable salt thereof alone or monotherapy with the anti-PD-1 antibody or antigen-binding fragment thereof alone. In some embodiments, administration of at least one cycle of the pharmaceutical combination of the present invention results in an increase in PFS of patients by at least about 1 month, about 2 months, about 3 months, about 4 months, about 5 months, about 6 months, about 7 months, about 8 months, about 9 months, about 10 months, about 11 months, about 1 year, about 2 years, or longer, compared with patients administered monotherapy with the anti-Nectin4 antibody-drug conjugate or pharmaceutically acceptable salt thereof alone or monotherapy with the anti-PD-1 antibody or antigen-binding fragment thereof alone. In some embodiments, administration of at least one cycle of the pharmaceutical combination of the present invention results in an increase in OS of patients by at least about 1 month, about 2 months, about 3 months, about 4 months, about 5 months, about 6 months, about 7 months, about 8 months, about 9 months, about 10 months, about 11 months, about 1 year, about 2 years, or longer, compared with patients administered monotherapy with the anti-Nectin4 antibody-drug conjugate or pharmaceutically acceptable salt thereof alone or monotherapy with the anti-PD-1 antibody or antigen-binding fragment thereof alone.

Compared with monotherapy with the anti-Nectin4 antibody-drug conjugate or pharmaceutically acceptable salt thereof alone or monotherapy with the anti-PD-1 antibody or antigen-binding fragment thereof alone, the pharmaceutical combination of the present invention results in an increased, preferably a synergistically increased, inhibition of tumor growth. In some embodiments, the pharmaceutical combination of the present invention results in tumor growth being inhibited by at least about 10%, about 20%, about 30%, about 40%, about 50%, about 60%, about 70%, or about 80%, compared with monotherapy with the anti-Nectin4 antibody-drug conjugate or pharmaceutically acceptable salt thereof alone or monotherapy with the anti-PD-1 antibody or antigen-binding fragment thereof alone. In some embodiments, administration of the pharmaceutical combination of the present invention results in an increased tumor regression, tumor shrinkage, and/or disappearance. In some embodiments, compared with untreated individuals or compared with monotherapy with the anti-Nectin4 antibody-drug conjugate or pharmaceutically acceptable salt thereof alone or monotherapy with the anti-PD-1 antibody or antigen-binding fragment thereof alone, the pharmaceutical combination of the present invention prevents tumor recurrence and/or increases survival duration in individuals, for example, increases survival duration by more than 15 days, more than 1 month, more than 3 months, more than 6 months, more than 12 months, more than 18 months, more than 24 months, more than 36 months, or more than 48 months. In some embodiments, the pharmaceutical combination of the present invention may increase progression-free survival or overall survival.

In some embodiments, administration of the pharmaceutical combination of the present invention to an individual with cancer results in a complete disappearance of the tumor ("complete response"). In some embodiments, administration of the pharmaceutical combination of the present invention to an individual with cancer results in a reduction of tumor cells or tumor size by at least 30% or more ("partial response"). Tumor reduction may be measured by any method known in the art, such as X-ray, positron emission tomography (PET), computed tomography (CT), magnetic resonance imaging (MRI), cytology, histology, or molecular genetic analysis.

In some embodiments, the pharmaceutical combination of the present invention can reduce adverse events caused by the administration of the anti-PD-1 antibodies or antigen-binding fragment thereof and/or anti-Nectin4 antibody-drug conjugates or pharmaceutically acceptable salt thereof alone, such as hematological toxicities, non-hematological toxicities, or other toxicities, e.g., pneumonia, diarrhea, renal insufficiency, rash, endocrine disorders, etc.

## IV. Kit of the present invention

Another objective of the present invention is to provide a kit of parts comprising the pharmaceutical combination of the present invention. Preferably, the kit is in a form of a pharmaceutical dosage unit. This allows the dosage unit to be provided according to a dosing regimen or drug administration interval.

In one embodiment, the kit of parts of the present invention comprises within a single package:

- A first container comprising a pharmaceutical composition for administration, wherein the pharmaceutical composition comprises an anti-Nectin4 antibody-drug conjugate or pharmaceutically acceptable salt thereof;
- A second container comprising a pharmaceutical composition for administration, wherein the pharmaceutical composition comprises an anti-PD-1 antibody or antigen-binding fragment thereof.

The various embodiments/technical solutions described herein and the features within each embodiment/technical solution should be understood as being capable of arbitrary combination with one another. Respective embodiments resulting from such combinations are encompassed within the scope of the present invention, as if each such combination were specifically and individually listed herein, unless the context clearly indicates otherwise.

## EXAMPLES

The following Examples provide a complete disclosure and description to those skilled in the art of how to prepare the composition of the present invention and the methods of use thereof, and are not intended to limit the scope of the present invention as perceived by the inventors.

### Example 1. Preparation of the anti-Nectin4 antibody-drug conjugate

According to the method described in Chinese Patent Application CN202210475286.3, the anti-Nectin4 antibody-drug conjugate (also abbreviated as "the anti-Nectin4-ADC") as shown below was prepared using hH2L1 (an anti-Nectin4 antibody) and a linker-payload (i.e., a linker-drug conjugate). The DAR was calculated via hydrophobic interaction chromatography (HIC): N=4.0.

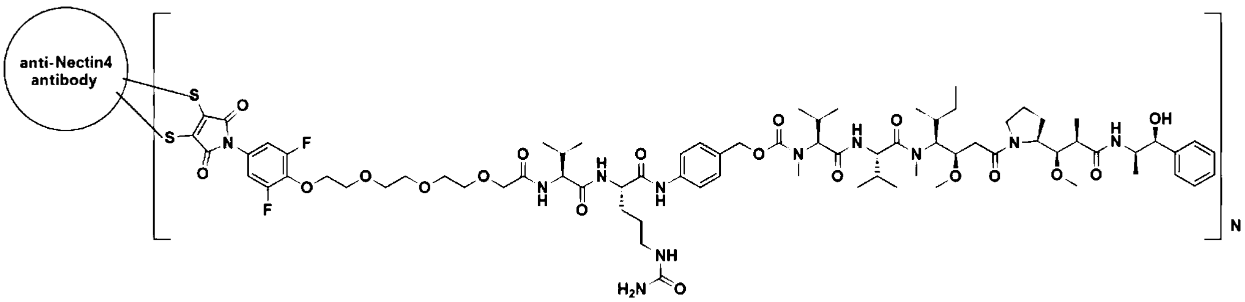

and/or

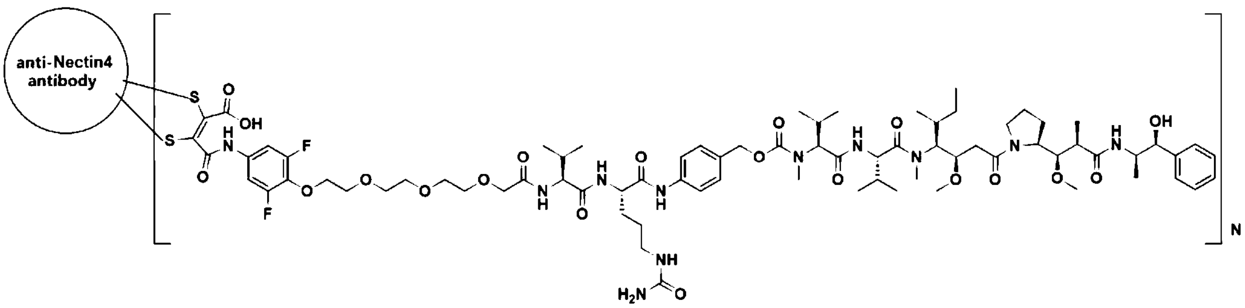

### Example 2. In vivo pharmaceutical efficacy of the anti-Nectin4-ADC combined with anti-PD-1 antibody on tumor-bearing mice - I

In this Example, tumor-bearing PD-1 HuGEMM-C57BL/6J mice were used as test animals to evaluate and compare the anti-tumor effects of the anti-Nectin4-ADC alone, anti-PD-1 antibody alone, or the combination of the anti-Nectin4-ADC and anti-PD-1 antibody on subcutaneously transplanted tumors in mice.

2.1 Test drugs and materials

2.1.1 Test drugs

Keytruda® (Lot No.: WO04692, purchased from MSD Ireland (Carlow)), an anti-PD-1 antibody pembrolizumab injection;

The anti-Nectin4-ADC: Prepared as described in Example 1.

2.1.2 Test animals

Mice: 6-8 week-old female PD-1 HuGEMM-C57BL/6J mice (purchased from Shanghai DiShi Biotechnology Co., Ltd.), in SPF (Specific Pathogen Free) grade

2.2 Experimental methods

Tumor-bearing PD-1 HuGEMM-C57BL/6J mice were prepared by inoculating PD-1 HuGEMM-C57BL/6J mice (6-8 weeks old, female, purchased from Shanghai DiShi Biotechnology Co., Ltd.) with MC38-hNECTIN4 mouse colon cancer cells.

Specifically, a tumor-bearing mouse model was established using MC38-hNECTIN4 mouse colon cancer cells (purchased from Shanghai Southern Model Biotechnology Co., Ltd.). MC38-hNECTIN4 cells were cultured in DMEM/High medium (comprising 10% inactivated fetal bovine serum) (at 37°C in a 5% $CO_2$ incubator). Logarithmic phase MC38-hNECTIN4 cells were resuspended in PBS to an appropriate concentration for subcutaneous tumor inoculation in mice. Using a 1 mL syringe, the cell suspension was inoculated subcutaneously into the right posterolateral body of PD-1 HuGEMM-C57BL/6J mice at 100 μL per mouse. Each mouse was inoculated with about $5.0 \times 10^6$ MC38-hNECTIN4 tumor cells, and a tumor-bearing PD-1 HuGEMM-C57BL/6J mouse model was established.

In this experiment, the anti-Nectin4-ADC (prepared with reference to Example 1) and the anti-PD-1 antibody Keytruda® (Lot No.: WO04692; purchased from MSD Ireland (Carlow)) were used for a grouped comparative study (as shown in Table 1). Grouping day was designated as Day 0. On grouping day, mice were given their first injection at a volume of 10μL/g. The solvent group was given an equivalent volume of the solvent (normal saline). Specific dosages and administration schedules were detailed in Table 1. Tumor volume was measured, mouse body weight was recorded, and data were documented twice weekly. At the end of the experiment, upon reaching the experimental endpoint, or when the tumor volume reached 3000 $mm^3$, the animals were euthanized by $CO_2$ anesthesia, followed by dissection, tumor removal, and photography.

Table 1. Dosages and administration schedules

| Group | Number of animals (n) | Group administered drug | Dose (mg/kg) | Concentration of administration (mg/ml) | Volume of administration (μL/g) | Route of administration | Dosing schedule |
|---|---|---|---|---|---|---|---|
| 1 | 8 | NS (normal saline) | - | - | 10 | i.v. | QW×2 doses (i.e., once weekly for a total of 2 doses) |
| 2 | 8 | The anti-Nectin4-ADC | 5 | 0.5 | 10 | i.v. | QW×2 doses |
| 3 | 8 | The anti-Nectin4-ADC | 10 | 1 | 10 | i.v. | QW×2 doses |
| 4 | 8 | Keytruda | 3 | 0.3 | 10 | i.p. | BIW× 4 doses (i.e., twice weekly for a total of 4 doses) |
| 5 | 8 | The anti-Nectin4-ADC | 2 | 0.5 | 10 | i.v. | QW×2 doses |
| | | Keytruda | 3 | 0.3 | 10 | i.p. | BIW× 4 doses |

(continued)

| Group | Number of animals (n) | Group administered drug | Dose (mg/kg) | Concentration of administration (mg/ml) | Volume of administration (μL/g) | Route of administration | Dosing schedule |
|---|---|---|---|---|---|---|---|
| 6 | 8 | The anti-Nectin4-ADC | 5 | 1 | 10 | i.v. | QW×2 doses |
| | | Keytruda | 3 | 0.3 | 10 | i.p. | BIW× 4 doses |

2.3 Experimental results and conclusions

Results were shown in Figure 1 and Table 2. Compared with the normal saline control group, no significant differences were observed in the groups administered a single drug alone. However, compared with the normal saline control group, the two groups administered the drug combination, i.e. the group administered the anti-Nectin4-ADC 2 mg/kg + Keytruda 3 mg/kg, and the group administered the anti-Nectin4-ADC 5 mg/kg + Keytruda 3 mg/kg, exhibited significant differences (p1 value < 0.05 and p1 value < 0.001, respectively).

The group administered the combination of the anti-Nectin4-ADC 2 mg/kg + Keytruda 3 mg/kg demonstrated a superior anti-tumor efficacy compared with the group administered Keytruda 3 mg/kg alone, and also showed significant differences compared with the group administered the anti-Nectin4-ADC 5 mg/kg alone, and the group administered the anti-Nectin4-ADC 10 mg/kg alone (p2 values < 0.01).

The group administered the combination of the anti-Nectin4-ADC 5 mg/kg + Keytruda 3 mg/kg demonstrated a superior anti-tumor efficacy compared with the group administered the anti-Nectin4-ADC 10 mg/kg alone and the group administered Keytruda 3 mg/kg alone, and also showed significant differences compared with each group administered the single drug alone (p3 values < 0.01). Although the total administered drug amount in the group administered the drug combination (the anti-Nectin4-ADC 5 mg/kg + Keytruda 3 mg/kg) was lower than that in the group administered the single drug alone (the anti-Nectin4-ADC 10 mg/kg alone), the former demonstrated a markedly superior pharmaceutical efficacy to the latter. This indicated that the drug combination of the anti-Nectin4-ADC and Keytruda exhibited a pronounced synergistic therapeutic effect.

In this experiment, no significant weight loss was observed in mice from the normal saline control group, the anti-Nectin4-ADC 5 mg/kg, the anti-Nectin4-ADC 10 mg/kg, Keytruda 3 mg/kg, the anti-Nectin4-ADC 2 mg/kg + Keytruda 3 mg/kg, and the anti-Nectin4-ADC 5 mg/kg + Keytruda 3 mg/kg groups (Figure 2). One mouse in Group 2 and one mouse in Group 4 died of tumor rupture on Day 15 and Day 19, respectively.

Table 2. Pharmaceutical efficacy analysis based on tumor volume in the subcutaneous mouse colon cancer model MC38-hNectin4 mice of each group

| Group | Administered drug | Dose (mg/kg) | Day 1 | Day 19 | | |
|---|---|---|---|---|---|---|
| | | | Tumor volume ($mm^3$) | Tumor volume ($mm^3$) | T/C (%) | TGI (%) |
| 1 | NS (normal saline) | - | 77.4±4.7 | 944.7±174.4 | - | - |
| 2 | the anti-Nectin4-ADC | 5 | 77.4±4.8 | 1172.9±163.5 | 126.3 | -26.3 |
| 3 | the anti-Nectin4-ADC | 10 | 77.4±4.4 | 667.7±141.6 | 68.1 | 31.9 |
| 4 | Keytruda | 3 | 77.3±4.4 | 623.2±152.0 | 62.9 | 37.1 |
| 5 | the anti-Nectin4-ADC + Keytruda | 2+3 | 77.3±4.5 | 460.9±111.2 | 44.2 | 55.8 |
| 6 | the anti-Nectin4 ADC+ Keytruda | 5+3 | 77.3±4.7 | 109.6±20.0 | 3.7 | 96.3 |
| Note: a. Tumor volume data were expressed as "mean ± standard error of the mean" (Mean ± SEM);<br>b. T/C % = T/C × 100%; TGI% =(1-T/C) × 100%, wherein Tand C represented respectively the absolute tumor volumes (Vt-V0) on Day 19 of the experiment for each treatment group and normal saline control group of the model. | | | | | | |

**Example 3. In vivo pharmaceutical efficacy of the anti-Nectin4-ADC combined with anti-PD-1 antibody on tumor-bearing mice - II**

In this Example, tumor-bearing PD-1-HU mice were used as test animals to evaluate and compare the anti-tumor effects of the anti-Nectin4-ADC alone, anti-PD-1 antibody alone, or the combination of the anti-Nectin4-ADC and anti-PD-1 antibody on subcutaneously transplanted tumors in mice.

3.1 Test drugs and materials

3.1.1 Test drugs

Tuoyi® (purchased from Junshi Biosciences), the main component of which is toripalimab, an anti-PD-1 antibody.
The anti-Nectin4-ADC: Prepared as described in Example 1.
PADCEV® (Enfortumab Vedotin): White lyophilized powder, 30mg/vial, Lot No. 102374, stored at 2-8°C protected from light. PADCEV® is an ADC drug conjugating a Nectin-4-targeting antibody to a microtubule inhibitor.

3.1.2 Test animals

Mice: 6 week-old female PD-1-HU mice (purchased from Shanghai Southern Model Biotechnology Co., Ltd.), in SPF grade

3.2 Experimental methods

Tumor-bearing PD-1-HU mice were prepared by inoculating PD-1-HU mice (6 weeks old, female, purchased from Shanghai Southern Model Biotechnology Co., Ltd.) with MC38/Nectin4 hPD-L1 mouse colon cancer cells.
Specifically, a tumor-bearing mouse model was established using MC38/Nectin4 hPD-L1 mouse colon cancer cells (purchased from Shanghai Southern Model Biotechnology Co., Ltd.). MC38/Nectin4 hPD-L1 cells were cultured in DMEM/High medium (comprising 10% inactivated fetal bovine serum) (at 37°C in a 5% $CO_2$ incubator). Logarithmic phase MC38/Nectin4 hPD-L1 cells were resuspended in PBS to an appropriate concentration for subcutaneous tumor inoculation in PD-1-HU mice. Using a 1 mL syringe, the cell suspension was inoculated subcutaneously into the right posterolateral body of PD-1-HU mice at 100 μL per mouse. Each mouse was inoculated with about 5.0 x $10^5$ MC38/Nectin4 hPD-L1 tumor cells, and a tumor-bearing PD-1-HU mouse model was established.
In this experiment, the anti-Nectin4-ADC and anti-PD-1 antibody Tuoyi (purchased from Junshi Biosciences) were used for a grouped comparative study (as shown in Table 3). After tumors grew to 80-100 $mm^3$ in tumor-bearing PD-1-HU mice, the animals were randomly assigned to groups based on tumor volume, with the grouping day designated as Day 0. The solvent group was given an equivalent volume of the solvent (normal saline). Specific dosages and administration schedules were detailed in Table 3. Tumor volume was measured, mouse body weight was recorded, and data were documented twice weekly. At the end of the experiment, upon reaching the experimental endpoint, or when the tumor volume reached 3000 $mm^3$, the animals were euthanized by $CO_2$ anesthesia, followed by dissection, tumor removal, and photography.

Table 3. Dosages and administration schedules

| Group | Number of Animals (n) | Group administered drug | Dose (mg/kg) | Route of administration | Time of dosing | Volume of administration (mL/kg) |
|---|---|---|---|---|---|---|
| 1 | 8 | solvent | normal saline | IP | D0, 7/ BIW×2 | 10 |
| 2 | 6 | the anti-Nectin4-ADC | 3 | IP | D0, 7/ BIW×2 | 10 |
| 3 | 6 | Tuoyi | 5 | IP | D0, 7/ BIW×2 | 10 |
| 4 | 6 | the anti-Nectin4-ADC+ Tuoyi | 3 mg/kg the anti-Nectin4-ADC, 5 mg/kg Tuoyi | IP | D0, 7/ BIW×2 | 10 |

(continued)

| Group | Number of Animals (n) | Group administered drug | Dose (mg/kg) | Route of administration | Time of dosing | Volume of administration (mL/kg) |
|---|---|---|---|---|---|---|
| 5 | 6 | PADCEV+ Tuoyi | 3 mg/kg PAD-CEV, 5 mg/kg Tuoyi | IP | D0, 7/ BIW×2 | 10 |
| Note: IP - intraperitoneal injection; dosing commenced on D0; BIW: twice weekly. | | | | | | |

Results were shown in Figures 3 and 4 and Table 4. Compared with the solvent control group, the group administered the single drug of the anti-Nectin4-ADC 3mg/kg alone, and the group administered the single drug of Tuoyi 5mg/kg alone, the two groups administered the drug combination, i.e. the group administered the anti-Nectin4-ADC 3 mg/kg + Tuoyi 5 mg/kg, and the group administered PADCEV 3 mg/kg + Tuoyi 5 mg/kg exhibited therapeutic effects to some degree on MC38/Nectin4 hPD-L1 subcutaneous grafted tumor. Compared with the group administered the single drug of Tuoyi alone, the two groups administered the drug combination, i.e. the group administered the anti-Nectin4-ADC 3 mg/kg + Tuoyi 5 mg/kg, and the group administered PADCEV 3 mg/kg + Tuoyi 5 mg/kg exhibited better anti-tumor effects. The group administered the drug combination of the anti-Nectin4-ADC 3 mg/kg + Tuoyi 5 mg/kg demonstrated a superior anti-tumor efficacy compared with the group administered the drug combination of PADCEV 3 mg/kg + Tuoyi 5 mg/kg. The group administered the anti-Nectin4-ADC 3 mg/kg + Tuoyi 5 mg/kg exhibited a superior anti-tumor efficacy compared with the group administered the single drug of the anti-Nectin4-ADC 3mg/kg alone. Tumor-bearing mice tolerated all drugs above well, with no significant weight loss or other symptoms observed. No animal deaths occurred during the experiment.

Table 4. Efficacy of the treatment on subcutaneously transplanted MC38/Nectin4 hPD-L1 mouse colon cancer

| Group | TV(D0) ($mm^3$) (Mean ± SEM) | TV(D14) ($mm^3$) (Mean ± SEM) | T/C(%) | TGI% |
|---|---|---|---|---|
| solvent | 89.1±0.9 | 2879±415.2 | - | - |
| the anti-Nectin4-ADC 3 mg/kg | 88.9±1 | 1823.8±779.9 | 62 | 38 |
| Tuoyi 5 mg/kg | 90.9±1.2 | 2045.5±596.7 | 70 | 30 |
| PADCEV 3 mg/kg+ Tuoyi 5 mg/kg | 88.9±0.7 | 1978.7±487.3 | 68 | 32 |
| the anti-Nectin4-ADC 3 mg/kg + Tuoyi 5 mg/kg | 89.6±1.2 | 1400.1±655.6 | 47 | 53 |
| Note: TV: Tumor volume, $mm^3$. | | | | |

**Example 4. Treatment of urothelial carcinoma using the anti-Nectin4-ADC combined with anti-PD-1 antibody**

This study is an open-label, single-arm, multicenter Phase Ib/II trial in patients with locally advanced or metastatic urothelial carcinoma. The study comprises a screening phase for patient enrollment, a treatment phase lasting up to 2 years, and a follow-up phase for safety/effectiveness assessment.

**1. Objectives of the clinical study:**

The primary objective was to evaluate the safety and tolerability of the anti-Nectin4-ADC combined with toripalimab in patients with locally advanced or metastatic urothelial carcinoma. The secondary objectives included the following:

1) To assess the preliminary efficacy of the anti-Nectin4-ADC combined with toripalimab in patients with locally advanced or metastatic urothelial carcinoma;

2) To evaluate the pharmacokinetic (PK) characteristics of the anti-Nectin4-ADC;

3) To evaluate the immunogenicity of the anti-Nectin4-ADC;

4) To evaluate the relationship between the biomarkers Nectin-4 and PD-L1 in tumor tissue and efficacy or prognosis.

The study protocol and all amendments have been approved by the institutional review boards of all participating centers.

**2. Test drugs:**

The active components of the investigational drugs described in this Example were the anti-Nectin4-ADC and the anti-PD-1 antibody as follows.

The anti-Nectin4-ADC: Prepared according to Example 1, supplied at 20 mg/vial, which is a conjugate of an anti-Nectin-4 antibody and MMAE;

Anti-PD-1 antibody: Toripalimab injection, manufactured by Shanghai Junshi Biosciences Co., Ltd.

**3. Patient inclusion criteria**

1) All subjects or their legal guardians must personally sign a written informed consent form approved by the Institutional Review Board before initiating any screening procedures;
2) Aged 18-80 years (inclusive) at the time of signing the informed consent form, with no restriction on gender;
3) Eastern Cooperative Oncology Group (ECOG) score 0-1;
4) Histopathologically confirmed locally advanced or metastatic urothelial carcinoma (including bladder, ureter, renal pelvis, and urethra). Urothelial carcinoma with squamous differentiation, glandular differentiation, or mixed types was also eligible, and radical surgical resection was not feasible;
5) Previous treatment history for locally advanced or metastatic urothelial carcinoma: Previous standard treatment failure or no prior systemic treatment received;
6) Archival tumor tissue samples must be provided or a new tumor tissue biopsy must be performed;
7) Estimated survival time of no less than 12 weeks;
8) According to RECIST v1.1 criteria, at least one measurable lesion was present at the screening period; for subjects with a history of definitive radiotherapy, the measurable lesion must be outside the radiation field or have shown definitive progression after completion of radiotherapy;
9) Subjects must meet the following organ function criteria. If a recent blood transfusion or growth factor therapy has been administered, hematological tests must be performed ≥ 7 days after any growth factor therapy or ≥ 14 days after any blood transfusion:

- Absolute neutrophil count (ANC) ≥ 1.5 × $10^9$/L;
- Platelet count (PLT) ≥ 100 × $10^9$/L;
- Hemoglobin (Hb) ≥ 90 g/L;
- Alanine aminotransferase (ALT) and aspartate aminotransferase (AST) ≤ 3 × the upper limit of normal (ULN);
- Total bilirubin ≤ 1.5 × ULN (total bilirubin for subjects with Gilbert's syndrome should be < 3 × ULN);
- International Normalized Ratio (INR) ≤ 1.5 × ULN;
- Creatinine clearance rate ≥ 50 mL/min (calculated using the Cockcroft-Gault formula), or serum creatinine ≤ 1.5 × ULN.

10) Male or female subjects must meet any one of the following criteria:

- Infertile females, who are specifically defined as those having undergone hysterectomy, bilateral oophorectomy, bilateral tubal ligation, or postmenopausal;
- Females of childbearing potential with a negative serum pregnancy test within 7 days before the first dose, and who agree to use effective contraceptive methods, e.g., double-barrier contraception, condoms, oral or injectable contraceptives, or intrauterine devices, and the like, throughout the study period and within 180 days after the last dose;
- Male subjects who have undergone vasectomy or agree to use contraceptive measures during the study treatment period and within 180 days after the last study drug administration.

11) Ability to understand and comply with scheduled visits, treatment, laboratory tests, and other study procedures.

**Exclusion criteria:**

1) Received anti-tumor therapy e.g., chemotherapy, radiotherapy within 21 days prior to the first study drug administration;

2) Underwent major surgery (defined as requiring general anesthesia and >24 hours of hospitalization) within 28 days prior to the first study drug administration, except for minor surgeries deemed by the investigator to not affect trial participation (e.g., tooth extraction, puncture);

3) (Cohort expansion phase only) Previous use of PD-1, PD-L1, or PD-L2 inhibitors for locally advanced or metastatic urothelial carcinoma, including but not limited to pembrolizumab, atezolizumab, or tislelizumab, etc.;

4) Previous treatment with antibody-drug conjugates having conjugated MMAE, such as Enfortumab vedotin;

5) Persistent clinically significant toxicity (Grade 2 or higher, except for alopecia and hyperpigmentation) related to prior therapy (including systemic therapy, radiotherapy, or surgery);

6) Subjects with Grade 2 or higher peripheral neuropathy;

7) Subjects with poorly controlled blood glucose;

8) Subjects assessed by the investigator as having an increased risk of developing corneal disease prior to the first dose;

9) Subjects with clinically significant cardiovascular or cerebrovascular diseases within 6 months prior to the first study drug administration, including but not limited to: ① acute myocardial infarction; ② unstable angina pectoris; ③ cerebrovascular accident (except for asymptomatic lacunar infarction not requiring clinical intervention); ④ history of clinically significant ventricular arrhythmias (e.g., sustained ventricular tachycardia, ventricular fibrillation, torsades de pointes); ⑤ New York Heart Association (NYHA) Class III or IV congestive heart failure; ⑥ QTcF $\geq$ 470 ms (females) or $\geq$ 450 ms (males), or a history of congenital long QT syndrome or family history thereof; ⑦ Baseline left ventricular ejection fraction (LVEF) < 50% or severe ventricular wall motion abnormalities detected by echocardiography (ECHO); ⑧ Subjects with hypertension poorly controlled by medication; ⑨ Other arrhythmias deemed ineligible for this study by the investigator;

10) Active infections, including but not limited to: hepatitis B virus infection (requiring both HBsAg positivity and HBV-DNA > 500 IU/mL), hepatitis C virus infection (requiring both HCV-Ab positivity and HCV-RNA positivity), human immunodeficiency virus infection (HIV-Ab positive), active Mycobacterium tuberculosis infection, or other active infections requiring systemic treatment within 14 days prior to the first study drug administration (except for routine prophylactic anti-infective therapy);

11) Subjects with other severe or uncontrolled diseases that the investigator believes would make the subject

unsuitable for this clinical trial or may affect compliance with the study protocol, including but not limited to: ① severe respiratory diseases, such as severe interstitial lung disease, severe asthma, etc.; ② severe arteriovenous thromboembolic events, such as pulmonary embolism, deep vein thrombosis (excluding asymptomatic intermuscular vein thrombosis not requiring specific treatment); ③ massive pleural effusion or ascites accompanied by clinical symptoms or requiring symptomatic management;

12) Subjects with central nervous system (CNS) metastases and/or carcinomatous meningitis. Subjects who have received prior treatment for brain metastases may be considered for inclusion in this study provided that their disease has been stable for at least 3 months, no disease progression has been confirmed by imaging within 4 weeks prior to the first study dose, all neurological symptoms have fully resolved, there is no evidence of new or expanding brain metastases, and radiation therapy, surgery, or glucocorticoid therapy has been discontinued for at least 28 days prior to the first study dose. Carcinomatous meningitis should be excluded regardless of clinical stability;

13) Subjects with a history of allogeneic hematopoietic stem cell transplantation or solid organ transplantation;

14) Subjects with a history of substance abuse or psychiatric disorders that may affect trial compliance;

15) Subjects with known allergy or intolerance to the study drugs or any of their components;

16) Subjects who used P-glycoprotein (P-gp) inhibitors or inducers, or potent CYP3A4 inhibitors or inducers, within 14 days prior to the first study dose;

17) Subjects who used high-dose glucocorticoids (>10 mg/day prednisone or equivalents) or other immunosuppressive drugs within 14 days prior to the first study dose;

18) Subjects who inoculated with live vaccines within 28 days prior to the first study dose or planned administration of any live vaccines during the study period;

19) Subjects who was given/administered other investigational drugs or experimental medical devices within 28 days prior to the first study dose;

20) Subjects with active autoimmune disease requiring systemic treatment within 2 years prior to the first study dose;

21) Subjects with other malignant tumors within 3 years prior to the first study dose, except for cancers that have undergone radical treatment and are expected to be cured, such as basal cell carcinoma or squamous cell carcinoma of the skin, localized low-risk prostate cancer, papillary thyroid carcinoma, or any type of carcinoma in situ that has been treated by radical resection, such as carcinoma in situ of the cervix or ductal carcinoma in situ of the breast;

22) Other conditions deemed ineligible for this study by the investigator.

Patients meeting the above criteria were enrolled and received the anti-Nectin4-ADC combined with toripalimab therapy.

**4. Clinical administration regimen**

Enrolled subjects received intravenous infusion of the anti-Nectin4-ADC on Day 1 (D1) and Day 8 (D8) of each cycle, and intravenous infusion of toripalimab on D1 of each cycle, and each treatment cycle was 21 days. The anti-Nectin4-ADC was administered at 1.0 mg/kg and 1.25 mg/kg respectively during the dose-escalation phase, and at 1.25 mg/kg during the cohort expansion phase. Toripalimab was administered at a dose of 240 mg. All subjects received a combination therapy of the anti-Nectin4-ADC and toripalimab until disease progression, occurrence of intolerable toxicity, death, withdrawal of informed consent, loss to follow-up, or completion of 2 years of the combination therapy, whichever occurred first.

**5. Results and summary**

**Anti-tumor activity**

For the combination therapy, the following clinical results were obtained. A total of 15 subjects in the study

completed at least one post-baseline tumor assessment, and all target lesions of these subjects showed varying degrees of shrinkage. All patients evaluated as having a partial response (PR) received treatment in the group. Among these, 4 tumor-evaluable patients who had previously received platinum-containing chemotherapy and PD-1/L1 therapy received the anti-Nectin4-ADC (1.25 mg/kg) combined therapy in this study, demonstrating an ORR of 75% (3/4).

In the published study (NCT05216965/CTR20220106) on the anti-Nectin4-ADC monotherapy (1.25 mg/kg) in patients with locally advanced or metastatic urothelial carcinoma who had previously undergone platinum-containing chemotherapy and PD-1/L1 therapy, 37 patients were tumor-evaluable, demonstrating an ORR of 62% (23/37).

In the study (POLARIS-03, NCT03113266/CTR20170347) on toripalimab therapy in patients with locally advanced or metastatic urothelial carcinoma who had previously undergone platinum-containing chemotherapy, the ORR was 25.8% (39/151) among 151 patients.

**Safety evaluation**

In the anti-Nectin4-ADC (1.25 mg/kg) combined therapy group, the common TRAEs (with an incidence rate of ≥10% across all grades) included skin rash, anemia, elevated aspartate aminotransferase, elevated serum creatinine, elevated alanine aminotransferase, nausea, decreased white blood cell count, hypercholesterolemia, alopecia, decreased neutrophil count, constipation, hypertriglyceridemia, pruritus, decreased platelet count, proteinuria, fatigue, decreased appetite, and weight loss.

Only one out of all subjects experienced a dose reduction of the anti-Nectin4-ADC due to an adverse event. No TRAEs resulting in death occurred. These results indicated that the combination of the anti-Nectin4-ADC and anti-PD-1 antibody demonstrated a favorable efficacy against urothelial carcinoma.

Although certain representative embodiments and details have been illustrated for the purpose of describing the present invention, it will be apparent to those skilled in the art that various changes and modifications may be made without departing from the scope of the present invention. In this regard, the scope of the present invention is limited only by the following claims.

**Exemplary sequences**

| **Sequence Number** (SEQ ID NO:) | **Description** | **Amino Acid Sequence** |
|---|---|---|
| 1 | hH2L1 antibody and hL2H1mut1 antibody HCDR1 (Kabat numbering) | DYGVS |
| 2 | hH2L1 antibody and hL2H1mut1 antibody HCDR2 (Kabat numbering) | VIWGGGKIYYNSVLKS |
| 3 | hH2L1 antibody and hL2H1mut1 antibody HCDR3 (Kabat numbering) | QGGLLFYAMDY |
| 4 | hH2L1 antibody and hL2H1mut1 antibody LCDR1 (Kabat numbering) | KSSQSLLNTYSQKNYLA |
| 5 | hH2L1 antibody and hL2H1mut1 antibody LCDR2 (Kabat numbering) | FASTRES |

(continued)

| **Sequence Number** (SEQ ID NO:) | **Description** | **Amino Acid Sequence** |
|---|---|---|
| 6 | hH2L1 antibody and hL2H1mut1 antibody LCDR3 (Kabat numbering) | QQHYNTPFT |
| 7 | hH2L1 antibody heavy chain variable region (VH) | EVQLQESGPGLVKPSETLSLTCTVSGFSLIDYGVSWIRQPPGKGLEWIGVIWGGGKIYYNSVLKSRVTISKDNSKSQVSLKLSSVTAADTAVYYCAKQGGLLFYAMDYWGQGTLVTVSS |
| 8 | hH2L1 antibody light chain variable region (VL) | DIVMTQSPDSLAVSLGERATINCKSSQSLLNTYSQKNYLAWYQQKPGQPPKLLIYFASTRESGVPDRFSGSGSGTDFTLTISSLQAEDVAVYYCQQHYNTPFTFGGGTKVEIK |
| 9 | hL2H1mut1 antibody heavy chain variable region (VH) | EVQLQESGPGLVKPSETLSLTCTVSGFSLIDYGVSWIRQPPGKGLEWIGVIWGGGKIYYNSVLKSRVTISVDTSKNQFSLKLSSVTAADTAVYYCAKQGGLLFYAMDYWGQGTLVTVSS |
| 10 | hL2H1mut1 antibody light chain variable region (VL) | DIVMTQSPDSLAVSLGERATINCKSSQSLLNTYSQKNYLAWYQQKPGQSPKLLIYFASTRESGVPDRFSGSGSETDFTLTISSLQAEDLAVYFCQQHYNTPFTFGGGTKVEIK |
| 11 | toripalimab HCDR1 (Kabat numbering) | DYEMH |
| 12 | toripalimab HCDR2 (Kabat numbering) | VIESETGGTAYNQKFKG |
| 13 | toripalimab HCDR3 (Kabat numbering) | EGITTVATTYYWYFDV |
| 14 | toripalimab LCDR1 (Kabat numbering) | RSSQSIVHSNGNTYLE |
| 15 | toripalimab LCDR2 (Kabat numbering) | KVSNRFS |
| 16 | toripalimab LCDR3 (Kabat numbering) | FQGSHVPLT |
| 17 | toripalimab heavy chain variable region (VH) | QGQLVQSGAEVKKPGASVKVSCKASGYTFTDYEMHWVRQAPIHGLEWIGVIESETGGTAYNQKFKGRVTITADKSTSTAYMELSSLRSEDTAVYYCAREGITTVATTYYWYFDVWGQGTTVTVSS |

(continued)

| Sequence Number (SEQ ID NO:) | Description | Amino Acid Sequence |
|---|---|---|
| 18 | toripalimab light chain variable region (VL) | DVVMTQSPLSLPVTLGQPASISCRSSQSIVHSNGNTYLE WYLQKPGQSPQLLIYKVSNRFSGVPDRFSGSGSGTDFT LKISRVEAEDVGVYYCFQGSHVPLTFGQGTKLEIK |
| 19 | toripalimab heavy chain | QGQLVQSGAEVKKPGASVKVSCKASGYTFTDYEMHW VRQAPIHGLEWIGVIESETGGTAYNQKFKGRVTITADK STSTAYMELSSLRSEDTAVYYCAREGITTVATTYYWY FDVWGQGTTVTVSSASTKGPSVFPLAPCSRSTSESTAA LGCLVKDYFPEPVTVSWNSGALTSGVHTFPAVLQSSG LYSLSSVVTVPSSSLGTKTYTCNVDHKPSNTKVDKRV ESKYGPPCPPCPAPEFLGGPSVFLFPPKPKDTLMISRTPE VTCVVVDVSQEDPEVQFNWYVDGVEVHNAKTKPREE QFNSTYRVVSVLTVLHQDWLNGKEYKCKVSNKGLPS SIEKTISKAKGQPREPQVYTLPPSQEEMTKNQVSLTCL VKGFYPSDIAVEWESNGQPENNYKTTPPVLDSDGSFFL YSRLTVDKSRWQEGNVFSCSVMHEALHNHYTQKSLS LSLGK |
| 20 | toripalimab light chain | DVVMTQSPLSLPVTLGQPASISCRSSQSIVHSNGNTYLE WYLQKPGQSPQLLIYKVSNRFSGVPDRFSGSGSGTDFT LKISRVEAEDVGVYYCFQGSHVPLTFGQGTKLEIKRTV AAPSVFIFPPSDEQLKSGTASVVCLLNNFYPREAKVQW KVDNALQSGNSQESVTEQDSKDSTYSLSSTLTLSKAD YEKHKVYACEVTHQGLSSPVTKSFNRGEC |
| 21 | pembrolizumab HCDR1 (Kabat numbering) | NYYMY |
| 22 | pembrolizumab HCDR2 (Kabat numbering) | GINPSNGGTNFNEKFKN |
| 23 | pembrolizumab HCDR3 (Kabat numbering) | RDYRFDMGFDY |
| 24 | pembrolizumab LCDR1 (Kabat numbering) | RASKGVSTSGYSYLH |
| 25 | pembrolizumab LCDR2 (Kabat numbering) | LASYLES |

(continued)

| Sequence Number (SEQ ID NO:) | Description | Amino Acid Sequence |
|---|---|---|
| 26 | pembrolizumab LCDR3 (Kabat numbering) | QHSRDLPLT |
| 27 | pembrolizumab heavy chain variable region (VH) | QVQLVQSGVEVKKPGASVKVSCKASGYTFTNYYMY WVRQAPGQGLEWMGGINPSNGGTNFNEKFKNRVTLT TDSSTTTAYMELKSLQFDDTAVYYCARRDYRFDMGF DYWGQG |
| 28 | pembrolizumab light chain variable region (VL) | EIVLTQSPATLSLSPGERATLSCRASKGVSTSGYSYLH WYQQKPGQAPRLLIYLASYLESGVPARFSGSGSGTDFT LTISSLEPEDFAVYYCQHSRDLPLTFGGG |
| 29 | pembrolizumab heavy chain | QVQLVQSGVEVKKPGASVKVSCKASGYTFTNYYMY WVRQAPGQGLEWMGGINPSNGGTNFNEKFKNRVTLT TDSSTTTAYMELKSLQFDDTAVYYCARRDYRFDMGF DYWGQGTTVTVSTASTKGPSVFPLAPCSRSTSESTAAL GCLVKDYFPEPVTVSWNSGALTSGVHTFPAVLQSSGL YSLSSVVTVPSSNFGTQTYTCNVDHKPSNTKVDKTVE RKCCVECPPCPAPPVAGPSVFLFPPKPKDTLMISRTPEV TCVVVDVSHEDPEVQFNWYVDGVEVHNAKTKPREEQ FNSTFRVVSVLTVVHQDWLNGKEYKCKVSNKGLPAPI EKTISKTKGQPREPQVYTLPPSREEMTKNQVSLTCLVK GFYPSDIAVEWESNGQPENNYKTTPPMLDSDGSFFLYS KLTVDKSRWQQGNVFSCSVMHEALHNHYTQKSLSLS PGK |
| 30 | pembrolizumab light chain | EIVLTQSPATLSLSPGERATLSCRASKGVSTSGYSYLH WYQQKPGQAPRLLIYLASYLESGVPARFSGSGSGTDFT LTISSLEPEDFAVYYCQHSRDLPLTFGGGTKVEIKRTV AAPSVFIFPPSDEQLKSGTASVVCLLNNFYPREAKVQW KVDNALQSGNSQESVTEQDSKDSTYSLSSTLTLSKAD YEKHKVYACEVTHQGLSSPVTKSFNRGEC |

## Claims

1. A pharmaceutical combination comprising

(i) an anti-Nectin4 antibody-drug conjugate or a pharmaceutically acceptable salt thereof; and
(ii) an anti-PD-1 antibody or an antigen-binding fragment thereof.

**2.** The pharmaceutical combination according to claim 1, wherein the anti-PD-1 antibody or the antigen-binding fragment thereof comprises

(i) a heavy chain variable region and a light chain variable region, wherein the heavy chain variable region comprises HCDR1 shown in SEQ ID NO:11, HCDR2 shown in SEQ ID NO:12, and HCDR3 shown in SEQ ID NO:13; and the light chain variable region comprises LCDR1 shown in SEQ ID NO:14, LCDR2 shown in SEQ ID NO:15, and LCDR3 shown in SEQ ID NO:16; or
(ii) a heavy chain variable region and a light chain variable region, wherein the heavy chain variable region comprises HCDR1 shown in SEQ ID NO:21, HCDR2 shown in SEQ ID NO:22, and HCDR3 shown in SEQ ID NO:23; and the light chain variable region comprises LCDR1 shown in SEQ ID NO:24, LCDR2 shown in SEQ ID NO:25, and LCDR3 shown in SEQ ID NO:26;

Preferably, the anti-PD-1 antibody or the antigen-binding fragment thereof comprises

(i) a heavy chain variable region and a light chain variable region, wherein the heavy chain variable region comprises a sequence shown in SEQ ID NO:17 or a sequence having at least 90%, 91%, 92%, 93%, 94%, 95%, 96%, 97%, 98%, or 99% identity therewith, and the light chain variable region comprises a sequence shown in SEQ ID NO:18 or a sequence having at least 90%, 91%, 92%, 93%, 94%, 95%, 96%, 97%, 98%, or 99% identity therewith; or
(ii) a heavy chain variable region and a light chain variable region, wherein the heavy chain variable region comprises a sequence shown in SEQ ID NO:27 or a sequence having at least 90%, 91%, 92%, 93%, 94%, 95%, 96%, 97%, 98%, or 99% identity therewith, and the light chain variable region comprises a sequence shown in SEQ ID NO:28 or a sequence having at least 90%, 91%, 92%, 93%, 94%, 95%, 96%, 97%, 98%, or 99% identity therewith;

More preferably, the anti-PD-1 antibody comprises

(i) a heavy chain sequence of SEQ ID NO:19 or a sequence having at least 90%, 95%, 98%, or 99% identity therewith, and a light chain sequence of SEQ ID NO:20 or a sequence having at least 90%, 95%, 98%, or 99% identity therewith; or
(ii) a heavy chain sequence of SEQ ID NO:29 or a sequence having at least 90%, 95%, 98%, or 99% identity therewith, and a light chain sequence of SEQ ID NO:30 or a sequence having at least 90%, 95%, 98%, or 99% identity therewith.

**3.** The pharmaceutical combination according to claim 1 or 2, wherein the anti-PD-1 antibody or the antigen-binding fragment thereof is an IgG antibody, preferably a human IgG antibody, more preferably a human IgG1 or human IgG4 antibody; and wherein the antigen-binding fragment is Fab, Fab', $F(ab')_2$, Fv, single-chain Fv, or single-chain Fab.

**4.** The pharmaceutical combination according to any one of claims 1 to 3, wherein the anti-Nectin4 antibody-drug conjugate or the pharmaceutically acceptable salt thereof has a molecular formula of Ab-[L-CTD]m, wherein Ab represents an anti-Nectin-4 antibody or an antigen-binding fragment thereof, L represents a linker, CTD represents a drug, and m represents an average number of drugs conjugated per Ab molecule; preferably, CTD is a cytotoxic drug; preferably CTD is one or more selected from the group consisting of: microtubule inhibitors MMAE, DM1, DM4, Tublysin, amanitin, calicheamicin, Eribulin and derivatives thereof; topoisomerase inhibitors SN38, Exatecan and derivatives thereof; and DNA binding agents PBD, doxorubicin and derivatives thereof; preferably, m is 1.0 to 5.0, preferably 3.0 to 4.2, more preferably 3.5 to 4.5, still more preferably 3.8 to 4.2, yet more preferably 3.9 to 4.1, and particularly preferably 4.0.

**5.** The pharmaceutical combination according to claim 4, wherein the anti-Nectin4 antibody-drug conjugate or the pharmaceutically acceptable salt thereof has a molecular formula of Ab-[L-CTD]m, wherein Ab represents an anti-Nectin-4 antibody or an antigen-binding fragment thereof, comprising a heavy chain variable region and a light chain variable region, wherein the heavy chain variable region comprises HCDR1 shown in SEQ ID NO:1, HCDR2 shown in SEQ ID NO:2, and HCDR3 shown in SEQ ID NO:3; and the light chain variable region comprises LCDR1 shown in SEQ ID NO:4, LCDR2 shown in SEQ ID NO:5, and LCDR3 shown in SEQ ID NO:6;

Preferably, the Ab comprises a heavy chain variable region and a light chain variable region, wherein the heavy chain variable region comprises a sequence shown in SEQ ID NO: 7 or a sequence having at least 90%, 91%, 92%, 93%, 94%, 95%, 96%, 97%, 98%, or 99% identity therewith; and the light chain variable region comprises a

sequence shown in SEQ ID NO: 8 or a sequence having at least 90%, 91%, 92%, 93%, 94%, 95%, 96%, 97%, 98%, or 99% identity therewith;
For example, the heavy chain variable region comprises an amino acid sequence shown in SEQ ID NO: 7 or SEQ ID NO: 9, and the light chain variable region comprises an amino acid sequence shown in SEQ ID NO: 8 or SEQ ID NO:10;
More preferably, the Ab comprises:

(i) a heavy chain variable region amino acid sequence shown in SEQ ID NO: 7; and a light chain variable region amino acid sequence shown in SEQ ID NO: 8; or
(ii) a heavy chain variable region amino acid sequence shown in SEQ ID NO: 9; and a light chain variable region amino acid sequence shown in SEQ ID NO:10;

Preferably, the Ab is an IgG antibody, more preferably a human IgG antibody, most preferably a human IgG1 or human IgG4 antibody; and wherein the antigen-binding fragment is Fab, Fab', $F(ab')_2$, Fv, single-chain Fv, or single-chain Fab.

**6.** The pharmaceutical combination according to any one of claims 1 to 5, wherein the anti-Nectin4 antibody-drug conjugate or the pharmaceutically acceptable salt thereof has a structure shown in Formula Ia and/or Ib below:

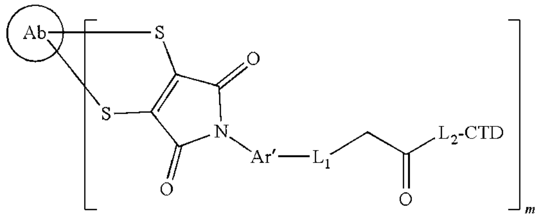

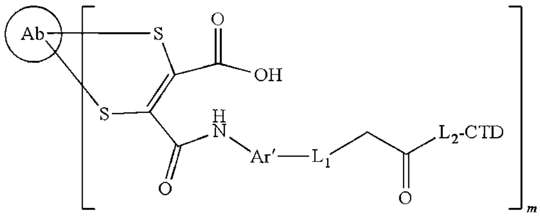

Ia and/or Ib

wherein: Ab is an anti-Nectin-4 antibody or an antigen-binding fragment thereof;
Ar' is any one selected from the group consisting of: substituted or unsubstituted C6-C10 arylene and substituted or unsubstituted 5-12 membered heteroarylene, wherein the substitution refers to the replacement of a hydrogen atom on a group by one or more substituents selected from the group consisting of: halogen (F, Cl, Br or I), halogenated alkyl (e.g. halogenated C1-C6 alkyl, preferably halogenated C1-C4 alkyl, e.g. trifluoromethyl) and alkoxy (e.g. C1-C6 alkoxy, preferably C1-C4 alkoxy, e.g. methoxy);
$L_1$ is $-O(CH_2CH_2O)n-$ linked to Ar', wherein n is any integer selected from the group consisting of 1 to 24, preferably any integer from 1 to 10, more preferably any integer from 3 to 5;
$L_2$ is an enzyme cleavable fragment, e.g., a dipeptide or a tripeptide or a tetrapeptide or a combination thereof with a self-immolating structural fragment (i.e., a polypeptide fragment consisting of 2-4 amino acids, or a combination of the polypeptide fragment with a self-immolating structural fragment), such as Val-Ala, Val-Ala-PAB, Val-Cit, Val-Cit-PAB, Phe-Lys-PAB, Ala-Ala-Ala, Gly-Gly-Phe-Gly (GGFG), MAC glucuronide phenol.

**7.** The pharmaceutical combination according to claim 6, wherein $L_2$-CTD is VcMMAE, GGFG-Dxd, or VC-seco-DUBA; preferably, if Ar' is a substituted or unsubstituted 5-12 membered heteroarylene, then the heteroatom is N; preferably, Ar' is a substituted or unsubstituted C6 arylene, or a substituted or unsubstituted 6 membered heteroarylene; more preferably, the antibody-drug conjugate or the pharmaceutically acceptable salt thereof has the following structure:

Conjugate ADC-1:

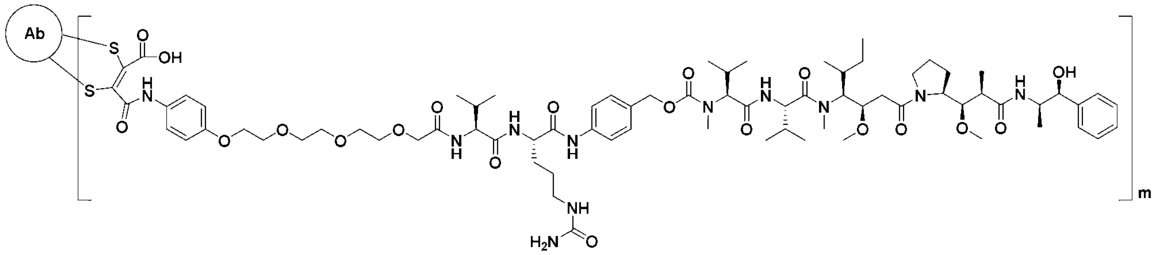

Conjugate ADC-2:

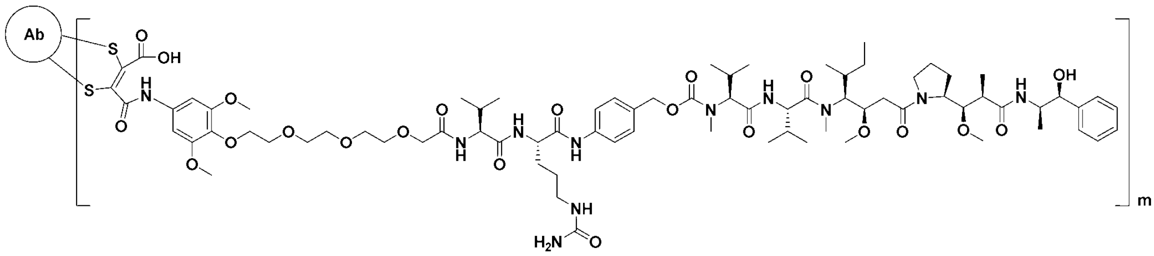

Conjugate ADC-3:

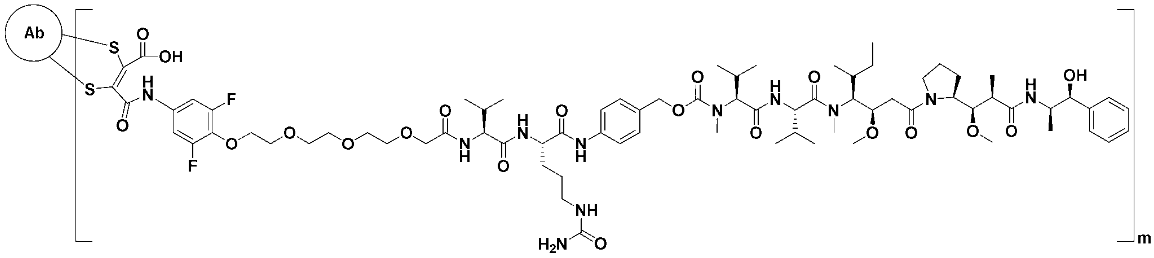

Conjugate ADC-4:

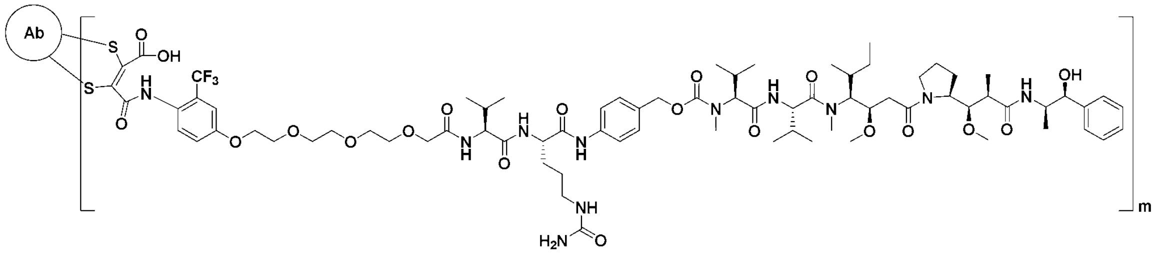

Conjugate ADC-5:

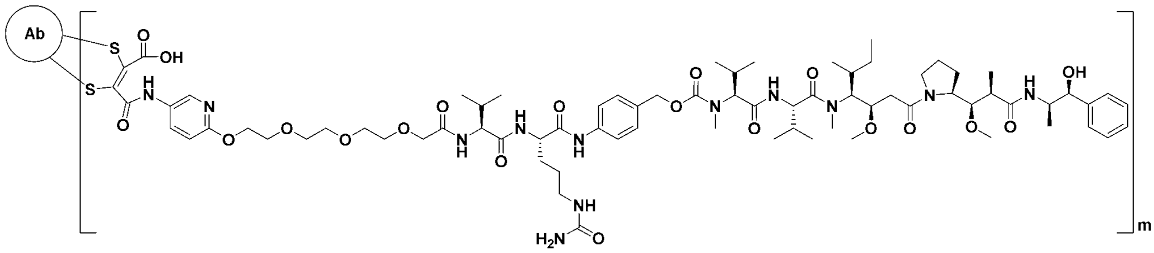

Conjugate ADC-6:

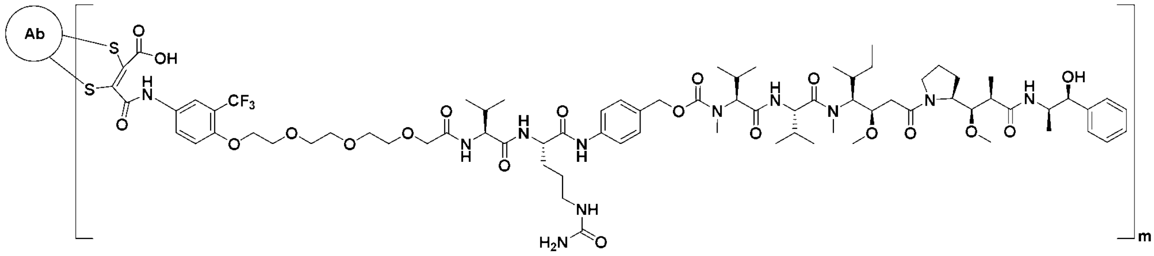

Conjugate ADC-7:

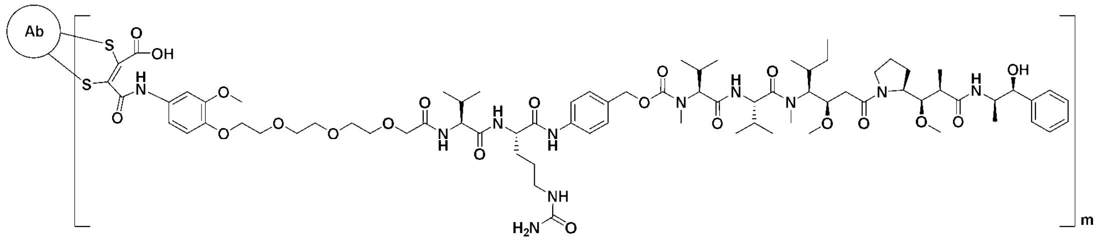

.

**8.** The pharmaceutical combination according to claim 7, wherein,

(i) an anti-Nectin4 antibody-drug conjugate or a pharmaceutically acceptable salt thereof is represented by the following formula:

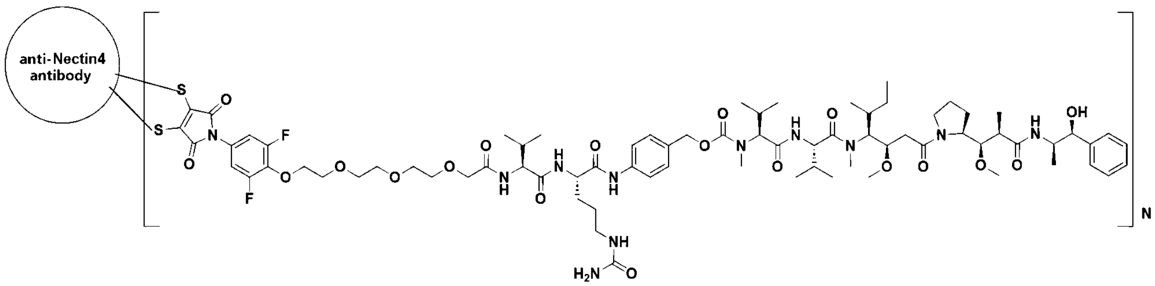

and/or

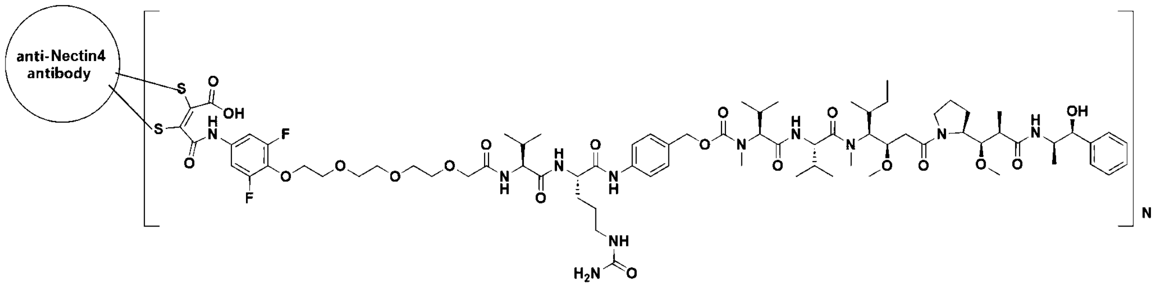

Wherein, the anti-Nectin4 antibody is as defined in claim 5,
N represents an average number of drugs conjugated per anti-Nectin-4 antibody molecule, and is 1.0 to 5.0, preferably 3.0 to 4.2, more preferably 3.5 to 4.5, still more preferably 3.8 to 4.2, yet more preferably 3.9 to 4.1, and particularly preferably 4.0; and

(ii) an anti-PD-1 antibody or an antigen-binding fragment thereof is selected from toripalimab or pembrolizumab, or an antigen-binding fragment thereof.

**9.** Use of the pharmaceutical combination according to any one of claims 1 to 8 for the manufacture of a medicament for preventing or treating a cancer, preferably the cancer is any one selected from the group consisting of: urothelial carcinoma, bladder cancer, breast cancer, ovarian cancer, pancreatic cancer, hepatocellular cancer, gastric cancer, non-hodgkin's lymphoma, hodgkin's lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, prostate cancer, colorectal cancer, colon cancer, glioma, and mesothelioma.

**10.** A kit of parts comprising the pharmaceutical composition according to any one of claims 1 to 8, preferably, the kit is in a form of a pharmaceutical dosage unit.

Effect of administered drugs on tumor volume in tumor-bearing PD-1 HuGEMM-C57BL/6J mice

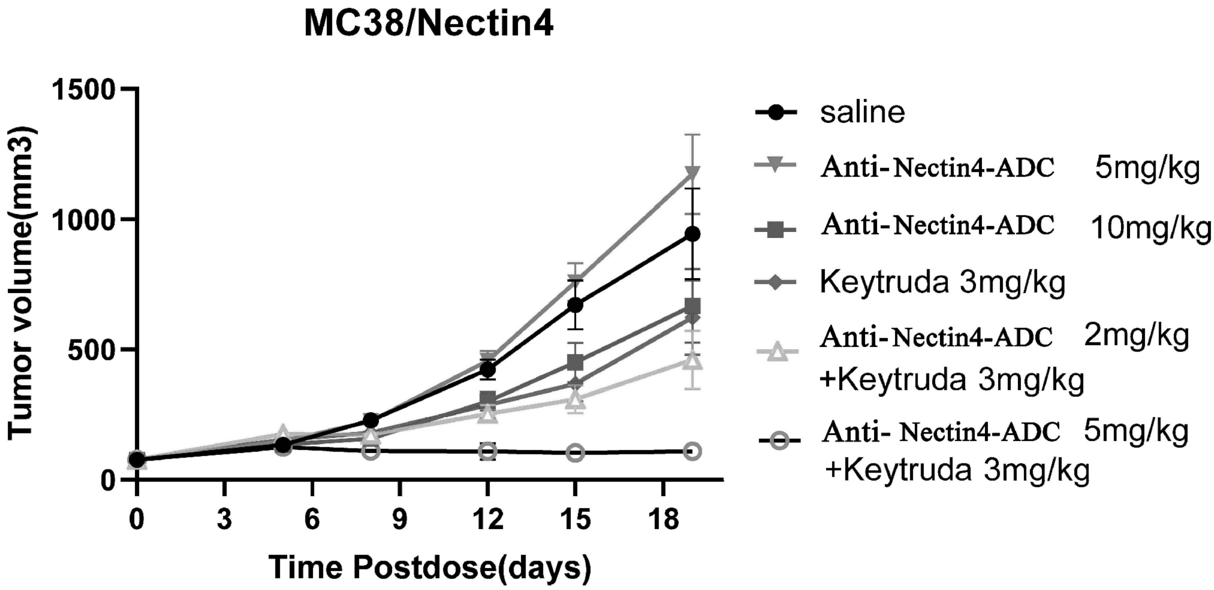


**Figure 1**

Effect of administered drugs on body weight in tumor-bearing PD-1 HuGEMM-C57BL/6J mice

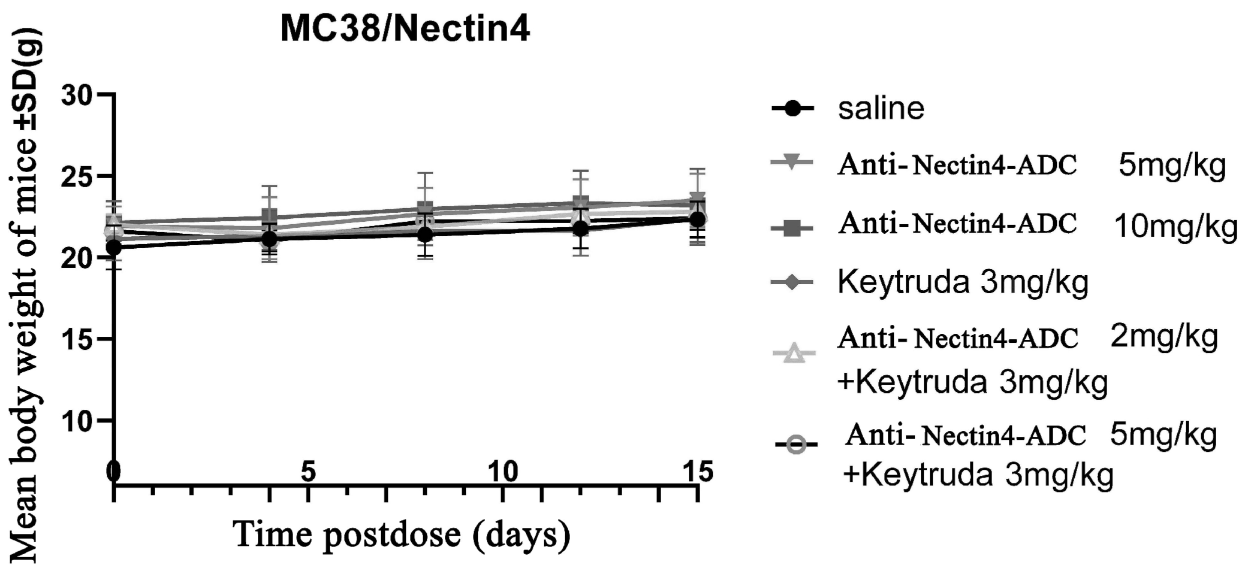


**Figure 2**

Effect of administered drugs on tumor volume in tumor-bearing PD-1-HU mice

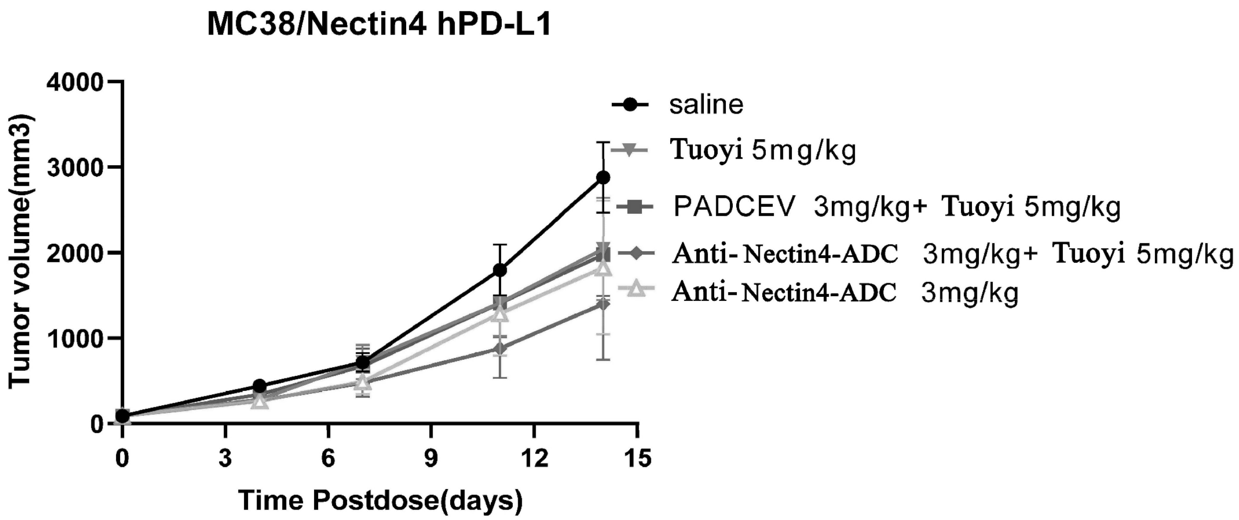


Figure 3

Effect of administered drugs on body weight in tumor-bearing PD-1-HU mice

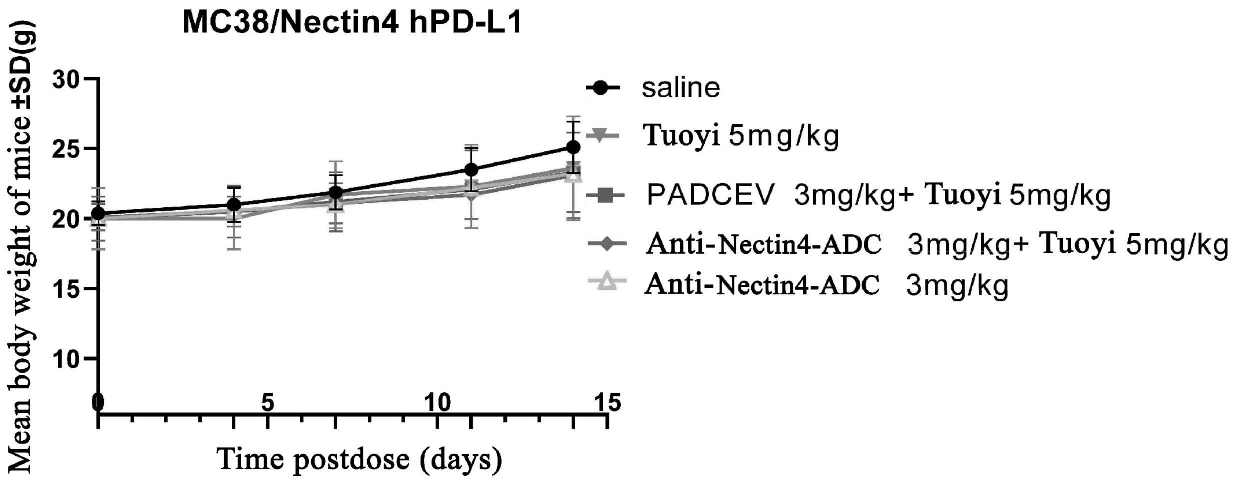


Figure 4

**INTERNATIONAL SEARCH REPORT**

International application No.
**PCT/CN2024/106818**

**A. CLASSIFICATION OF SUBJECT MATTER**

A61K45/06(2006.01)i； A61K47/68(2017.01)i； A61K39/395(2006.01)i； A61K39/00(2006.01)i； A61P35/00(2006.01)i

According to International Patent Classification (IPC) or to both national classification and IPC

**B. FIELDS SEARCHED**

Minimum documentation searched (classification system followed by classification symbols)

IPC： A61K, A61P

Documentation searched other than minimum documentation to the extent that such documents are included in the fields searched

Electronic data base consulted during the international search (name of data base and, where practicable, search terms used)

CNABS; CNTXT; DWPI; ENTXT; ENTXTC; CNKI; STNext REGISTRY/CAPLUS: 抗体药物偶联物, 联用, ADC, combination, PD-1, Nectin-4, Enfortumab vedotin, pembrolizumab

**C. DOCUMENTS CONSIDERED TO BE RELEVANT**

| Category* | Citation of document, with indication, where appropriate, of the relevant passages | Relevant to claim No. |
|---|---|---|
| X | HOIMES, C. J. et al. "Enfortumab Vedotin Plus Pembrolizumab in Previously Untreated Advanced Urothelial Cancer" *Journal of Clinical Oncology,* Vol. 41, No. 1, 30 August 2022 (2022-08-30), 22-31 abstract | 1, 3, 9-10 |
| Y | HOIMES, C. J. et al. "Enfortumab Vedotin Plus Pembrolizumab in Previously Untreated Advanced Urothelial Cancer" *Journal of Clinical Oncology,* Vol. 41, No. 1, 30 August 2022 (2022-08-30), 22-31 abstract | 2-10 |
| Y | CN 113244386 A (SHANGHAI JUNSHI BIOSCIENCES CO., LTD.) 13 August 2021 (2021-08-13) claims 1-9 | 2-10 |
| Y | CN 115252813 A (IANGSU MABWELL HEALTH PHARMACEUTICAL R&D CO., LTD. et al.) 01 November 2022 (2022-11-01) claims 1-19, and description, paragraphs 0129-0133 | 4-10 |

☑ Further documents are listed in the continuation of Box C. ☑ See patent family annex.

* Special categories of cited documents:

"A" document defining the general state of the art which is not considered to be of particular relevance

"D" document cited by the applicant in the international application

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **23 October 2024** | **30 October 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/106818**

**C. DOCUMENTS CONSIDERED TO BE RELEVANT**

| Category* | Citation of document, with indication, where appropriate, of the relevant passages | Relevant to claim No. |
|---|---|---|
| X | O'DONNELL, Peter et al. "Enfortumab Vedotin With or Without Pembrolizumab in Cisplatin-Ineligible Patients With Previously Untreated Locally Advanced or Metastatic Urothelial Cancer" *Journal of Clinical Oncology,* Vol. 41, No. 25, 27 June 2023 (2023-06-27), 4107-4117<br>abstract | 1, 3, 9-10 |
| X | WO 2022060831 A1 (MIRATI THERAPEUTICS, INC.) 24 March 2022 (2022-03-24)<br>claims 1-4 | 1, 3, 9-10 |
| A | WO 2023133388 A2 (AGENSYS, INC. et al.) 13 July 2023 (2023-07-13)<br>entire document | 1-10 |

Form PCT/ISA/210 (second sheet) (July 2022)

**INTERNATIONAL SEARCH REPORT**

International application No.
**PCT/CN2024/106818**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/106818**

| Patent document cited in search report | | | Publication date (day/month/year) | Patent family member(s) | | | Publication date (day/month/year) |
|---|---|---|---|---|---|---|---|
| CN | 113244386 | A | 13 August 2021 | None | | | |
| CN | 115252813 | A | 01 November 2022 | US | 2024226318 | A1 | 11 July 2024 |
| | | | | JP | 2024517776 | A | 23 April 2024 |
| | | | | KR | 20240004708 | A | 11 January 2024 |
| | | | | EP | 4331615 | A1 | 06 March 2024 |
| | | | | CA | 3217112 | A1 | 03 November 2022 |
| | | | | AU | 2022267613 | A1 | 23 November 2023 |
| | | | | MX | 2023012861 | A | 08 December 2023 |
| | | | | BR | 112023022549 | A2 | 02 January 2024 |
| | | | | WO | 2022228563 | A1 | 03 November 2022 |
| WO | 2022060831 | A1 | 24 March 2022 | WO | 2022060831 | A8 | 27 October 2022 |
| | | | | US | 2023321062 | A1 | 12 October 2023 |
| | | | | EP | 4213844 | A1 | 26 July 2023 |
| WO | 2023133388 | A2 | 13 July 2023 | WO | 2023133388 | A3 | 21 September 2023 |
| | | | | TW | 202333801 | A | 01 September 2023 |
| | | | | IL | 313915 | A | 01 August 2024 |
| | | | | AU | 2023205782 | A1 | 27 June 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8609089 B
- US 20150079109 A
- WO 2015085847 A
- WO 2015112800 A
- WO 2015112900 A
- WO 2014179664 A
- WO 2014194302 A
- CN 202210475286


### Non-patent literature cited in the description


- **YAO S** ; **ZHU Y** ; **CHEN L**. Advances in targeting cell surface signaling molecules for immune modulation. *Nat. Rev. Drug Discov.*, 2013, vol. 12 (2), 130-146
- **CHOTHIA et al.** Canonical structures for the hypervariable regions of immunoglobulins. *Journal of Molecular Biology*, 1987, vol. 196, 901-917
- **KABAT et al.** Sequences of Proteins of Immunological Interest. U.S. Department of Health and Human Services, National Institutes of Health, 1987
- **NORTH et al.** A New Clustering of Antibody CDR Loop Conformations. *Journal of Molecular Biology*, 2011, vol. 406, 228-256
- Fundamental Immunology. Raven Press, 1993
- **ESTEP, P et al.** High throughput solution-based measurement of antibody-antigen affinity and epitope binning. MAbs, 2013, vol. 5, 270-278
- **NEEDLEMA-WUNSCH**. *J. Mol. Biol.*, 1970, vol. 48, 444-453
- **E. MEYERS** ; **W. MILLER**. *CABIOS*, 1989, vol. 4, 11-17
- **PARDOLL DM.** The blockade of immune checkpoints in cancer immunotherapy. *Nat Rev Cancer*, 2012, vol. 12 (4), 252-264
- **YAO S** ; **ZHU Y** ; **CHEN L**. Advances in targeting cell surface signaling molecules for immune modulation. *Nat Rev Drug Discov*, 2013, vol. 12 (2), 130-146
- **PIA M** ; **CHALLITA-EID et al.** *Cancer Res.*, 2016, vol. 76 (10), 3003-13